# EUROPEAN PATENT SPECIFICATION

(11) **EP 4 000 681 B1**
(45) Date of publication and mention of the grant of the patent: **29.01.2025**
(21) Application number: 21305824.1
(22) Date of filing: 16.06.2021
(51) Int. Cl.: A61N 1/05

(54) **ACTIVE FIXATION LEAD**
AKTIVES FIXIERKABEL
DÉRIVATION À FIXATION ACTIVE

(30) Priority: 16.11.2020 FR 2011721
(43) Date of publication of application: 25.05.2022
(73) Proprietor: Sorin CRM SAS, 92140 Clamart (FR)
(72) Inventor: Ollivier, Jean-François, 91190 Gif sur Yvette (FR)
(74) Representative: Ungerer, Olaf

(56) References cited:
- US-A- 5 354 327
- US-A1- 2010 331 943
- US-A1- 2013 245 732
- US-A1- 2017 239 464
- US-B1- 6 687 550

## Description

### Field of Invention

The present invention relates to an implantable lead configured to be connected to a pacemaker or a defibrillator and comprising an active fixation for securing an electrode to cardiac tissue.

### Background of the invention

It is known that active fixation leads comprise an extendable and retractable mechanism for deploying a helix electrode at a distal end of the lead. In the known extendable and retractable mechanism, the helix electrode is electrically connected to and supported by an electrically conductive driver shaft movably disposed within a distal housing portion of the lead. The helix electrode is thus electrically connected to a distal coil conductor of the lead by means of the conductive driver. The helix electrode could be in intermittent contact with an internal wall of the distal housing portion of the lead in which is it movably housed.

The distal housing portion of the lead is usually a cylindrical molded plastic part. However, the distal housing portion of the lead may be provided with metallic part(s).

The presence of metal part in the distal housing portion of the lead involves that if the helix electrode and the metallic part of the housing are not continuously electrically connected, electrical chatter noises may be generated. Indeed, chatter noise may result from the intermittent contacts of these metallic parts of the lead, especially as the helix electrode or the distal metallic part of the housing is in contact with blood, being an ionic solution. Chatter noise is a parasite electrical signal that can cause the delivery of inappropriate therapy or shock.

For addressing the electrical chatter noise problem occurring between a metallic housing collar of the lead and the helix electrode, the document US 6,687,550 B1discloses the use of a compression metallic coil spring or a "ball seal" type contact spring. According to the document US 6,687,550 B1, such spring is positioned around the conductive driver, thereby generating an electrical contact between the spring and the driver, the driver being electrically connected to the helix electrode and the distal coil conductor of the lead. In particular, in the document US 6,687,550 B1, a garter spring (i.e. a circular spring) is housed in the annular recess of an annular track member disposed between the driver and a tubular conductive coupling. The annular track member according to the document US 6,687,550 B1 allows radially compressing the garter spring over the entire periphery of the driver.

However, the radial compression of the garter spring over the entire periphery of the driver increases the friction between the spring and the driver, which may generate parasitic mechanical effects. Indeed, the mechanical friction between the spring and the driver can affect the smoothness of the driver's movement, and makes the driver moves in a jerky manner. Such parasitic friction will thus jeopardize the quality of the helix screw deployment.

Further examples of prior-art stimulation leads are known from US 2017/239464 A1 and US 2010/331943 A1.

Hence, a first object of the present invention is to improve the prevention of chatter noise in an active fixation lead without negatively affecting the quality of the helix screw deployment.

Moreover, in the known pacing and/or defibrillation lead, the distal housing portion of the lead comprising the retractable fixation mechanism and an anode provided at the distal end of the lead constitutes the most rigid portion of the distal part of the lead. In particular, there is the type of lead wherein said "rigid" portion is a one-piece portion completely rigid and about 14 to 16 mm long. There is also a type of lead wherein said "rigid" portion actually comprises two distinct rigid sections separated by a more flexible portion having a length between 1 and 3 mm.

The presence of a flexible portion, as mentioned above with respect to the second type of lead, is generally preferred by clinicians because it allows facilitating the implantation of the lead, in particular in the neighboring of the heart chambers and for passing the tricuspid valve. On the other hand, because the short flexible portion (having a length of 1 to 3 mm) is arranged between two more rigid portions of the lead, it may impact the long-term reliability of the lead. Indeed, the mechanical stress generated by the cyclic cardiac contractions is mostly absorbed by the short flexible section of 1 and 3 mm length.

Hence, a second object of the present invention is to provide a pacing and/or defibrillation lead comprising a retractable fixation mechanism wherein the rigid distal portion of the lead has a reduced length compared to that of the leads from the state of the art, in order to facilitate its placement.

There is also a need for improving the sealing properties of the known pacing and/or defibrillation lead wherein the distal housing portion of the lead comprises the retractable fixation mechanism. Indeed, fluid, like blood, can enter the lumen of the distal housing portion of the lead via the distal opening through which the fixation mechanism can be extended and retracted. As blood is an ionic solution, surface and/or volume corrosion can occur inside the distal end of the lead.

Hence, a third object of the invention is to improve the sealing properties of the distal end portion of an active fixation lead and, in particular, provide as sealing solution that allows an easy assembly of the lead.

### Description of the Invention

The present invention provides an implantable pacing and/or defibrillation lead according to claim 1**.** The dependent claims define embodiments of the present invention.

Said lead extending from a proximal end to a distal end, wherein the proximal end of the lead is configured to be connected to an implantable medical device, the lead comprising: - an active fixation electrode at the distal end of the lead, which is mechanically and electrically coupled to a conductive driver shaft moveably arranged in a distal housing portion of the lead, wherein the active fixation electrode is movable from a retracted position in which the active fixation electrode is completely housed inside the distal housing, to an extended position, in which the active fixation electrode extends in a distal direction at least partially beyond the distal housing portion, and - an electrically conductive compression spring. According to the present invention the lead further comprises a guiding member arranged inside the distal housing portion and comprising a tubular body electrically connected with the distal housing portion, wherein the conductive driver shaft is slideably arranged through a lumen extending through the tubular body, wherein the tubular body further comprises a radial opening in communication with the lumen, and wherein the electrically conductive compression spring is partially radially arranged around the tubular body and its radial opening such that the electrically conductive compression spring is forced by its restoring force against the conductive driver shaft.

Hence, the occurrence of chatter noise can be avoided by means of a continuous and stable electrical contact provided between the active fixation electrode and the distal housing portion that brings the active fixation electrode and the distal housing portion onto the same electrical potential. The continuous electrical contact is realized by the electrically conductive compression spring forced by its restoring force against the conductive driver shaft.

The spring provides multi-point contacts with the conductive driver shaft at the radial opening by means of the spring's individual coils. As the electrically conductive compression spring can only contact the conductive driver shaft at the radial opening of the tubular body, friction between the electrically conductive compression spring and the conductive driver shaft may only occur at the radial opening, and not over the entire periphery of the conductive driver shaft. Thus, in comparison with the state of the art, friction can be advantageously reduced. As less unwanted mechanical friction is generated than in the state of the art, the quality of the active fixation electrode is improved and a smooth deployment of the active fixation electrode can be achieved.

For sake of completeness, it is noted that the expression "conductive driver shaft" refers to an electrically conductive driver shaft.

The implantable pacing and/or defibrillation lead of claim 1 can be further improved according to various advantageous embodiments.

According to one embodiment, the electrically conductive compression spring can be a closed coiled helical spring.

It allows advantageously reducing the size of the spring while facilitating the assembly of the lead as the closed coiled helical spring can be maintained around the tubular body without the need of additional retaining means. The successive coils of the spring provide multi-point contacts with the conductive driver shaft at the radial opening, therefore ensuring a redundancy of the electrical contacts.

According to one embodiment, the electrically conductive compression spring can be an open coiled helical spring comprising two free-ends.

Hence, the solution for avoiding chatter noise according to the present invention can advantageously be implemented with different type of springs.

According to one embodiment, at least a portion of the tubular body provided with the radial opening can have an outer radius greater than an inner radius of the electrically conductive compression spring at its rest state.

The radial dimension difference causes the spring to be forced against the conductive driver shaft by its restoring force and thus ensures the electrical contact.

According to one embodiment, the radial opening can have an opening angle comprised between 100° and 250°, in particular between 150° and 210°, in a cross section of the tubular body perpendicular to a direction of extension of the lead.

The dimension of the radial opening is adapted to provide an opening allowing the spring to generate a restoring force sufficient to provide a stable contact against the conductive driver shaft.

According to one embodiment, the radial opening can have a width along a direction of extension of the lead greater than the extension of the electrically conductive compression spring in that direction.

Hence, sufficient space is provided to reliably bring the spring into contact with the conductive driver shaft as the spring can freely retract onto the driver shaft when positioned in the radial opening.

According to one embodiment, the inner diameter of the distal housing portion can be larger than the diameter of the electrically conductive compression spring partially radially arranged around the tubular body and its radial opening.

Hence, in the lead, because of their dimension's differences, the spring is not compressed by the distal housing portion, which would lead to unwanted larger friction.

According to one embodiment, the tubular body can have at least one chamfered edge at the radial opening.

The presence of chamfered edge prevents damaging the spring, in comparison with sharp edge. Thereby, the robustness and the lifetime of the lead can be improved.

According to one embodiment, the tubular body can comprise at least - a first portion provided with the radial opening and a portion of the tubular body contacting the distal housing portion, and - a second portion extending from a distal end of the tubular body to the first portion, and wherein a sealing means can be provided at the second portion to seal the first portion of the tubular body from the distal end of the lead.

Hence, the first portion of the tubular body, which comprises the spring, can be advantageously sealed against fluids like blood, being an ionic solution. It allows preventing surface and/or volume corrosion, in particular at the electrically conductive compression spring.

According to one embodiment, the tubular body of the guiding member can be mechanically and electrically connected to the distal housing portion by means of a welding, and/or by means of a crimping means and/or a heat shrinked means arranged to maintain the tubular body in electrical and mechanical contact with the distal housing.

It further allows providing a lead easy to assemble, thereby improving the manufacturing efficiency
It further allows establishing a stable electrical connection between the tubular body of the guiding member and the distal housing portion.

The first object of the invention is further achieved by means the method according to claim 11 for assembling an implantable pacing and/or defibrillation lead according to one of claims 1-10. Said method comprises the steps of: a) providing the electrically conductive compression spring around the tubular body of the guiding member at the radial opening such that a portion of the electrically conductive compression spring is partially deflected inside the lumen due to its restoring force, then b) inserting the conductive driver shaft through the lumen of the conductive guiding member thereby pushing at least partially the compression spring outside the lumen so as to provide an electrical connection between the electrically conductive compression spring and the conductive driver shaft, and then c) mechanically and electrically connecting the tubular body of the guiding member to the distal housing portion of the lead.

According to one embodiment, step b) can further comprise providing a sealing means at the distal end of the tubular body to seal it from the distal end of the lead.

It allows sealing the tubular body from the distal end of the lead against fluids like blood, being an ionic solution. It thus allows preventing surface and/or volume corrosion, in particular at the electrically conductive compression spring.

According to one embodiment, step d) can comprise welding the tubular body to the distal housing portion of the lead, in particular by laser welding.

It allows establishing a stable electrical connection between the tubular body of the guiding member and the distal housing portion.

The present disclosure also relates to an exemplary pacing and/or defibrillation lead comprising a lead body provided with: a proximal end configured to be connected to an implantable medical device, and a distal end, opposite the proximal end, the distal end of the lead body comprising an annular electrode forming an anode of said lead, the lead further comprising a sleeve in which an electrode forming a cathode of the lead is at least partially housed, in particular said electrode is a retractable fixation screw, wherein the sleeve is partially accommodated in the distal end of the lead body such that the annular electrode of the lead body is superimposed to the sleeve.

It is noted that the above-mentioned sleeve is a distal housing of portion of the lead.

The ring electrode of the lead body is superimposed to the sleeve. In other words, the ring electrode is arranged above the sleeve in a radial direction of the lead.

As a result, the rigid portion of the lead is advantageously compacted axially, i.e. along the longitudinal axis of the lead, which allows the length of the rigid portion to be reduced compared to the known leads. In fact, two functions of the lead are superimposed because the annular electrode, which has an anode function for detection and/or stimulation is superimposed, i.e. arranged above, the sleeve comprising an electrode, which has a cathode function for detection and/or stimulation. The electrodes constitute so-called "rigid" portions of the lead. In the case of leads with active fixation, the sleeve comprises an electrode in the form of a retractable screw, which has a combined function of cathode for detection and/or stimulation and means of fixation to a tissue. The structure of the lead thus allows a plurality of lead functions to be arranged in a way that the length of the rigid portion of the lead can be reduced (i.e. along the longitudinal axis of the lead), thereby facilitating its implantation and placement within the body of a patient.

It also allows avoiding the presence of a more flexible portion between two rigid portions, as known from the leads according to the state of the art. Thus, the lead according to the present invention is more reliable and robust over time.

The exemplary implantable pacing and/or defibrillation lead can be further improved according to various advantageous embodiments.

According to one embodiment, the sleeve can be electrically insulated from the annular electrode of the lead body by an electrically insulating sheath disposed on the sleeve, in particular a sheath heat-shrunk (i.e. heat-retracted) onto the sleeve.

The electrically insulating sheath is arranged between the ring electrode and the sleeve in a radial direction of the lead. The ring electrode is thus superimposed to the sleeve, so that the ring electrode is arranged above the sleeve, but the ring electrode is not directly arranged on the sleeve.

The electrically insulating sleeve allows the isolation of the anode pole from the cathode pole. The use of a sheath on the sleeve has the advantage of being a compact structural solution.

In addition, the thermo-shrink formation avoids the presence of an air gap between the sleeve and the sheath and can easily be adapted to any shape.

According to an embodiment, the electrically insulating sheath can be a Teflon sheath shrunk onto the sleeve.

The use of Teflon has the technical effect of improving the electrical insulation performance of the sheath because the efficiency of Teflon (tetra-fluoroethylene) in terms of electrical insulation is particularly satisfactory.

According to one embodiment, the sleeve can be made of a metallic material.

By using a metallic material for the sleeve, the wall thickness of the sleeve can be reduced in comparison to the thickness obtained by using a plastic material. Thus, either the diameter of the sleeve can be advantageously reduced, or the internal space of the sleeve can be enlarged, thus allowing the insertion of a fastening screw of larger diameter.

According to one embodiment, a proximal end of the sleeve can be accommodated within the distal end of the lead body and can comprise a shoulder.

The shoulder of the proximal end of the sleeve provides an axial stop on which the anode of the lead body can rest and thus mechanically hold the sleeve to the lead body.

According to one embodiment, the ring electrode can be attached to the sleeve by clamping or crimping.

Thus, the proximal end of the sleeve with the shoulder can be mechanically held to the lead body without the need for welding. This simplifies the assembly of the lead.

According to one embodiment, the ring electrode can be clamped or crimped in at least two distinct areas of the anode.

Thus, it is not necessary to crimp or clamp the ring electrode around its entire circumference to ensure the mechanical connection between the sleeve and the lead body. This further simplifies the assembly while ensuring a reliable mechanical connection overtime.

According to one embodiment, the cardiac stimulation lead can further comprise a retaining ring welded to the ring electrode of the distal end of the lead body and disposed, along a radial direction of the lead, at least partially between the ring electrode and the sleeve, the retaining ring can further comprise a shoulder at one end thereof, said shoulder being disposed, along a longitudinal direction of the lead, between the ring electrode and the sleeve.

The use of a retaining ring offers an alternative way of securing the sleeve to the lead body.

According to one embodiment, the annular electrode can have a surface of at least 15 square millimeters.

This anode surface allows preserving sensing functions compatible with atrial and ventricular positioning.

According to one embodiment, the lead can comprise an electrically insulating adhesive, in particular silicone adhesive or glue, between the distal end of the lead body and the proximal end of the sleeve accommodated in the distal end of the lead body.

Thus, the electrical isolation between the cathode and anode can be further improved, as the adhesive provides an additional sealing barrier.

In addition, the introduction of glue minimizes any relative movement between the sleeve and the distal end of the lead body.

The present disclosure also relates to an exemplary method for assembling the exemplary stimulation lead according to one of the above mentioned embodiments. Said method comprises at least the steps of 1) thermoforming an electrically insulating sheath over a portion of the sleeve, and 2) accommodating said portion of the sleeve within the distal end of the lead body so that the annular electrode of the lead body is superimposed on the sleeve.

As a result, the rigid portion of the lead is advantageously compacted axially (i.e. along the longitudinal axis of the lead), which makes it possible to reduce the length of the rigid portion compared to known leads.

In fact, two functions of the lead are superimposed because the annular electrode, which has an anode function for detection and/or stimulation is superimposed to, i.e. arranged above, the sleeve comprising an electrode which has a cathode function for detection and/or stimulation. The electrodes constitute so-called "rigid" portions of the lead. In the case of leads with active fixation, the sleeve comprises an electrode in the form of a retractable screw, which has a combined function of cathode and means of fixation to a tissue. The structure of the lead thus allows a plurality of functions of the lead to be arranged in a way to reduce the length (i.e., along the longitudinal axis of the lead) of the rigid portion of the lead, thereby facilitating its implantation and placement within the body of a patient.

It allows also avoiding the presence of a more flexible portion between two rigid portions, as known from the lead of the state of the art. Thus, the lead according to the present invention is more reliable and robust over time.

The electrically insulating sleeve allows the insulation of the anode pole (i.e. the ring electrode) from the cathode pole (i.e. the electrode in the sleeve). The use of a sheath on the sleeve has the advantage of being a compact structural solution.

In addition, the thermo-shrink formation allows avoiding the presence of an air gap between the sleeve and the sheath and can easily be adapted to any shape.

According to one embodiment, the method can further comprise a step of clamping or crimping the ring electrode to the sleeve.

Thus, the proximal end of the sleeve with the shoulder can be mechanically held to the lead body without the need for welding. This simplifies the assembly of the lead.

According to one embodiment, step 2) of the method can further comprise: welding a retaining ring to the ring electrode of the lead body, and inserting the sleeve into the lead body such that the retaining ring is, in a radial direction of the lead, at least partially between the ring electrode and the sleeve, and such that a shoulder provided at one of the ends of the retaining ring is arranged, in a longitudinal direction of the lead, between the ring electrode and the sleeve.

The use of a retaining ring provides an alternative way of securing the sleeve to the lead body.

According to one embodiment, the method can further comprise a step of introducing electrically insulating adhesive between the distal end of the lead body and the portion of the sleeve accommodated in the distal end of the lead body.

Thus, the electrical isolation between the cathode and anode can be further improved, as the adhesive provides an additional sealing barrier.

In addition, the introduction of glue allows minimizing any relative movement between the sleeve and the distal end of the lead body.

The present invention also provides an implantable pacing and/or defibrillation lead according to claim 14.

Hence, the dimensions of the sealing ring of the sealing means allows avoiding fluidic communication from the distal end of the lead wherein blood can enter the distal housing portion. It thus allows at least partially sealing the distal housing portion, in particular wherein metallic parts may be housed, such as an electrically conductive spring for providing an electrical connection. The sealing means thus decrease the risk of surface and/or volume corrosion, in particular between a fluid and such an electrically conductive spring.

This is achieved thanks to the dimensions of the sealing ring, the first portion of which having dimensions allowing a rotation and a translation of the conductive driver shaft within the sealing ring while forming a seal at an interface between the conductive driver shaft and the sealing ring.

It is further achieved thanks to the dimensions of the second portion of the sealing ring forming a seal at an interface between the internal distal housing portion and the sealing ring.

As the first portion of the sealing ring have an external diameter strictly less than an internal diameter of the distal housing portion, there is no stress applied on the sealing ring by the distal housing portion at the first portion of the sealing ring. Hence, at the first portion, the sealing ring is only deformed, in particular elastically deformed, at the interface between the first portion and the conductive driver shaft.

It allows preserving a good functionality of the retraction and extension mechanism while avoiding blood clot within functional gap between mechanical parts of the lead.

According to one embodiment, the percentage difference of diameter between the external diameter of the first portion of the sealing ring and the internal diameter of the distal housing portion can be comprised between 3 % and 30%, particular between 5% and 20%.

Hence, it allows reducing the friction between the sealing ring and the distal housing portion, so as to not hinder the rotation and the translation of the conductive driver shaft with respect to the sealing means at the first portion of the sealing ring. By avoiding friction, the active fixation electrode can be deployed more smoothly, without unwanted jerking motion of the conductive driver shaft.

Such range can render a fluidic communication possible at the interface between the distal housing portion and the first portion of the sealing ring. The sealing properties are however ensured at the interface between the conductive driver shaft and the first portion of the sealing means, and at the interface between the distal housing portion and the second portion of the sealing means.

According to one embodiment, the percentage difference between the internal diameter of the first portion of the sealing ring and an external diameter of the conductive driver shaft be comprised between 0,05 % and 20%, in particular between 1% to 10%.

The above-mentioned range of percentage difference allows the conductive driver shaft to rotate and slide with respect to the sealing ring while preventing fluid communication at the interface between the conductive driver shaft and the first portion of the sealing ring.

According to one embodiment, the percentage difference between the external diameter of the second portion of the sealing ring and the internal diameter of the distal housing portion can be comprised between 5 % and 20%, in particular between 1% and 10%.

The above-mentioned range of percentage difference allows forming a seal and preventing fluid communication at the interface between the distal housing portion and the second portion of the sealing ring.

According to one embodiment, the internal diameter of the first portion of the sealing ring can be less than the internal diameter of the second portion of the sealing ring.

It allows providing a lead wherein the dimension of the first portion is adapted for receiving the conductive driver shaft while the dimension of the second portion is adapted for receiving the tubular body wherein the conductive driver shaft is slideably and rotatably arranged through the lumen of the tubular body.

The form-fitted connection allows locking the seal means to the tubular body, in particular a friction-locked connection.

According to one embodiment, the internal radial recess of the sealing ring can be provided at a junction between the first portion and the second portion of the sealing ring.

Hence, a mechanically stable retention by the form-fitted connection can be obtained.

According to one embodiment, the first portion of the sealing ring can have a rounded edge along its internal diameter.

It allows reducing the friction generated between the sealing ring and the distal housing portion, so as to not hinder the rotation and the translation of the conductive driver shaft with respect to the sealing means at the first portion of the sealing ring.

According to one embodiment, the sealing means can be integrally formed in one-piece.

Hence, the sealing means can be easily manufacturing and assembled to the lead, thereby reducing the assembly time and the assembly cost.

According to one embodiment, the sealing means can be made of rubber, in particular of silicone, and can have a Shore hardness comprised between 50 to 65 Shore A.

The stress-strain behavior of a rubber sealing means can be linked to its Shore hardness. The selection of the Shore hardness in the range 50-65 Shore A allows ensuring that a sufficient compressive force is exerted on the flexible rubber sealing means in the distal housing portion. The Shore hardness of the rubber sealing means is thus adapted to provide the sealing properties in the arrangement of the sealing means inside the distal end housing.

Additional features and advantages will be described with reference to the drawing. In the description, reference is made to the accompanying figure that is meant to illustrate embodiments of the invention. It is understood that such embodiments do not represent the full scope of the present invention which is defined by the appended claims.
**Figure 1** represents a cross-sectional view of a cardiac stimulation lead according to a first embodiment of the invention.
**Figure 2** represents a cross-sectional view of the cardiac pacing lead according to a second embodiment of the invention.
**Figure 3A** depicts a cross-sectional view of a first step for the cardiac pacing lead according to the first embodiment of the invention.
**Figure 3B** represents a cross-sectional view of a second step for assembling the cardiac pacing lead according to the first embodiment of the invention.
**Figure 3C** depicts a cross-sectional view of a third step for assembling the cardiac pacing lead according to the first embodiment of the invention.
**Figure 3D** represents a cross-sectional view of a fourth step for assembling the cardiac pacing lead according to the first embodiment of the invention.
**Figure 3E** depicts a cross-sectional view of a fifth step for assembling the cardiac pacing lead according to the first embodiment of the invention.
**Figure 3F** represents a schematic view of the fifth step for assembling the cardiac pacing lead according to the first embodiment of the invention.
**Figure 3G** depicts a cross-sectional view of a sixth step for assembling the cardiac pacing lead according to the first embodiment of the invention.
**Figure 4A** represents a cross-sectional view of a step for assembling the cardiac pacing lead according to the second embodiment of the invention, prior to adhesive or glue deposition.
**Figure 4B** represents a cross-sectional view of a step for assembling the cardiac pacing lead according to the second embodiment of the invention, after adhesive or glue deposition.
**Figure 5** illustrates a cross-sectional view of a lead's distal end parallel to a direction of extension of the lead according to a third embodiment of the present invention.
**Figure 6A** and **Figures 6B** illustrate schematic views of a guiding member according to the third embodiment of the present invention.
**Figure 7** illustrates a spring according to the third embodiment the present invention.
**Figure 8** illustrates a cross-sectional view perpendicular to a direction of extension of the lead of the guiding member and the spring according to the third embodiment of the present invention.
**Figure 9A** illustrates a schematic view of an assembly comprising a conductive driver shaft, the guiding member and the spring according to the third embodiment of the present invention.
**Figure 9B** illustrates a cross-sectional view perpendicular to a direction of extension of the lead of Figure 9A.
**Figure 10** illustrates a schematic view of an assembly comprising a helix screw, a sealing means, the conductive driver shaft, the guiding member and the spring according to the third embodiment of the present invention.
**Figure 11** illustrates a cross-sectional view of a lead's distal end parallel to a direction of extension of a lead according to a fourth embodiment of the present invention.
**Figure 12** illustrates a cross-sectional view of a sealing means of the lead according to the first, the second and the third embodiments of the present invention.
**Figure 13** illustrates a cross-sectional view of the distal end of lead according to the first, the second and the third embodiments of the present invention, comprising the sealing means represented in Figure 12.

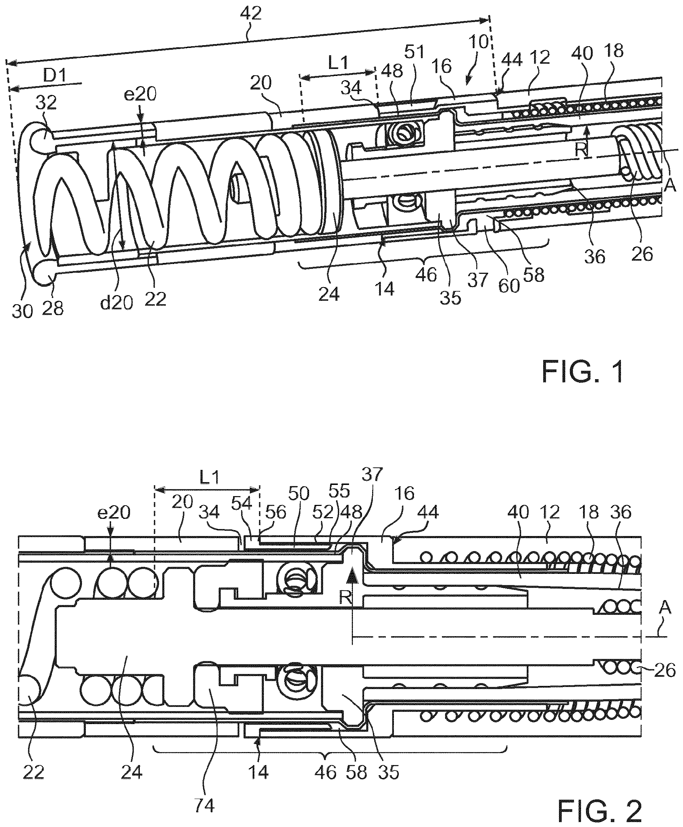

**Figure 1** depicts a cross-sectional view of a cardiac pacing lead 10 according to a first embodiment of the invention.

The pacing lead 10 shown in Figure 1 is a retractable screw lead. In one embodiment, it may be a defibrillation lead.

In another embodiment, it may be a passive fixation lead comprising barbs arranged radially around the lead body with a stimulation electrode at the distal end.

The lead 10 comprises a substantially cylindrical lead body 12 that extends longitudinally along an axis A, which constitutes an axis of revolution.

The lead body 12 comprises a proximal end (not shown in Figure 1) configured for connection to an implantable medical device (not shown in Figure 1) and a distal end 14 that is opposite said proximal end.

The distal end 14 of the lead body 12 comprises an annular electrode 16. The annular electrode 16 forms an anode of the lead 10. The annular electrode 16 and the lead body 12 have substantially the same diameter.

As shown in Figure 1, the distal end 14 of the lead body 12 is terminated by the ring electrode 16.

Between the distal end 14 and the proximal end of the lead 10, coiled electrical conductors 18 are housed within the lead body 12. In particular, the electrical conductors 18 allow the ring electrode 16, i.e., the anode, to be electrically connected to one pole of the implantable medical device connector (not shown in Figure 1).

The lead 10 further comprises a sleeve 20 in which a retractable fixation screw 22 is housed. The retractable attachment screw 22 forms a cathode of the lead 10. The retractable fixation screw 22 is a helical screw made of conductive material, connected through a metal tip 24, i.e. an electrically conductive driver shaft 24, to an internal conductor 26. The coiled conductor 26 provides electrical continuity between the retractable fixation screw 22 (which acts as a sensing and stimulation electrode) and a generator located at the proximal end (not shown in Figure 1) of the lead 10.

The sleeve 20 is substantially cylindrical in shape with the same diameter as the ring electrode 16 and the lead body 12.

The sleeve 20 comprises a distal end 28 provided with an opening 30 dimensioned such that the retractable fixation screw 22 is configured to be deployed along a direction D1 out of the sleeve 20 through said opening 30. The opening 30 in the distal end 28 is provided with an edge 32 around the entire circumference of the sleeve 20. The edge 32 is substantially rounded and therefore is not sharp or cutting. A sealing means 74 (visible in Fig. 1 but only annotated in Fig. 2) is provided in the sleeve 20 to at least partially seal the lead 10 from the distal end 28.

The sleeve 20 comprises, opposite to its distal end 28, a proximal end 34.

At the proximal end 34, a mouthpiece 35 of the lead 10 is bordered around its entire circumference by a shoulder 37.

The sleeve 20 is partially accommodated at its proximal end 34 within the distal end 14 of the lead body 12 such that the annular electrode 16 of the lead body 12 is superimposed on the sleeve 20, i.e., the annular electrode 16 is disposed above the sleeve 20 along a radial direction R of the lead.

As it will be described below, the annular electrode 16 is not disposed directly on the sleeve 20 due to the presence of an electrically insulating sheath (shown by the reference sign 48) disposed along the radial direction R of the lead between the annular electrode 16 and the sleeve 20.

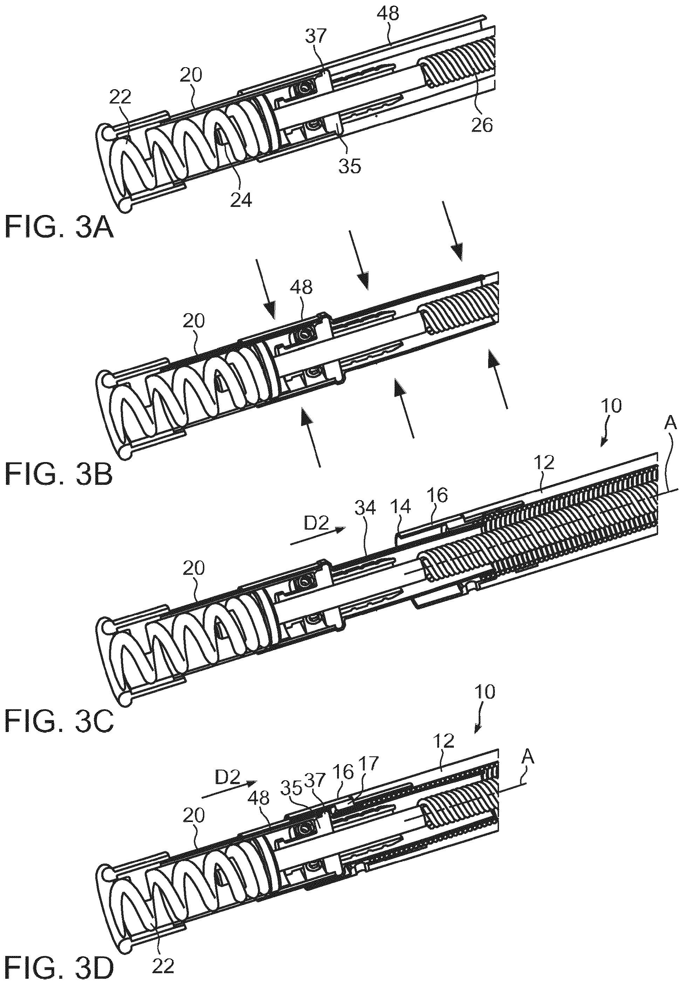

The structure and geometry of the sleeve 20 is further appreciated in the drawings of **Figures 3A-3C** described below, which illustrate the sleeve 20 outside the lead body 12.

In one embodiment wherein the lead is a passive fixation lead, the stimulation electrode may have a substantially solid cylinder shape and may be at least partially housed within the sleeve 20 such that the stimulation electrode, like the retractable fixation screw 22, extends along the axis A. The distal end of the stimulation electrode in such embodiment can be a substantially smooth, flat or curved surface configured to contact tissue. Such a lead is then held in place by means of barbs arranged radially around the lead body.

The metal tip 24, i.e. the electrically conductive driver shaft 24, and the inner conductor 26 are housed in the lead body 12 and are arranged, in particular partially arranged, within the circumference 36 defined by spiral electrical conductors 18.

Thus, two functions of the lead 10 are superimposed along a radial direction R of the lead: a portion of the anode 16 (sensing/stimulation function) is superimposed with the sleeve 20 comprising the retractable screw 22 (fixation and sensing/stimulation function). As a result, the rigid portion 42 of the lead 20, i.e., the portion 42 that extends from the distal end 28 of the sleeve 20 to the junction 44 between the annular electrode 16 and the lead body 12, is advantageously compacted axially along the axis A (i.e., along the longitudinal axis of the lead 10), thereby allowing reducing the length of the rigid portion 42 as compared to that of known leads. This also makes it possible to avoid the presence of a flexible portion between two rigid portions, as proposed by some leads known from the state of the art.

The sleeve 20 is partially accommodated in the distal end 14 of the lead body 12 such that the distance L1 between the cathode 22 and the anode 16 is at least 3 millimeters. This distance L1 allows to preserve sensing functions that are compatible with an atrial and ventricular implantation of the lead 10.

In addition, the anode 16 preferably has a surface area of at least 15 square millimeters to preserve sensing and pacing functions compatible with an atrial and ventricular implantation of the lead 10.

The sleeve 20 is made of a metallic material. The use of a metallic material for the sleeve 20 allows the thickness "e20" of the wall of the sleeve 20 to be reduced in comparison to the thickness involved by the use of a plastic material. Thus, either the diameter d20 of the sleeve 20 can be advantageously reduced, or the internal volume of the sleeve can be enlarged, thereby allowing the insertion of a fixation screw 22 of larger diameter.

The conductive elements of the sleeve 20, i.e., the retractable fixation screw 22, the electrically conductive driver shaft 24, and the inner conductor 26, are insulated from the conductive elements (i.e. the coiled electrical conductors 18) connected to the annular electrode 16 of the lead body 12 by a cylindrical tube 40 made of an electrically insulating material.

In addition, in order to electrically isolate the cathode (i.e. the retractable fixation screw 22) from the anode (i.e. the ring electrode 16) at the overlap area 46 of the sleeve 20 at the distal end 14 of the lead body 12, an electrically insulating sheath 48 is disposed on the sleeve 20. The electrically insulating sheath 48 is disposed between the annular electrode 16 and the sleeve 20 along the radial direction of the lead 10. The electrically insulating sheath 48 may be a Teflon sheath shrunk onto the sleeve 20 at the overlap area 46.

The electrically insulating sleeve 48 allows the isolation of the pole constituted by the anode 16 from the pole constituted by the cathode 22, in a radial direction R of the lead body 12. The use of a sheath 48 arranged on the sleeve 20 has the advantage of being a structurally compact solution. Indeed, the sheath 48 can be arranged in the residual space between the sleeve 20 and the lead body 12 superimposed on the sleeve 20, which is typically less than 50 microns.

In addition, the heat shrink formation eliminates the need for an air gap between the sleeve 20 and the sheath 48 and allows for easy adaptation to any shape.

Moreover, the efficiency of Teflon (tetra-fluoroethylene) in terms of electrical insulation is particularly satisfactory.

However, the use of a Teflon sheath 48 does not allow the use of known conventional adhesives such as silicone or polyurethane to ensure the mechanical connection between the sleeve 20 and the lead body 12, as Teflon constitutes a non-stick coating.

Therefore, as shown in Figure 1, the annular electrode 16 is deformed, by clamping or crimping, to form a recess 51. This recess 51 abuts on the shoulder 37 of the mouthpiece 35 of the sleeve 20. In other words, the shoulder 37 provides an axial stop for the anode 16 which mechanically holds the sleeve 20 to the lead body 12.

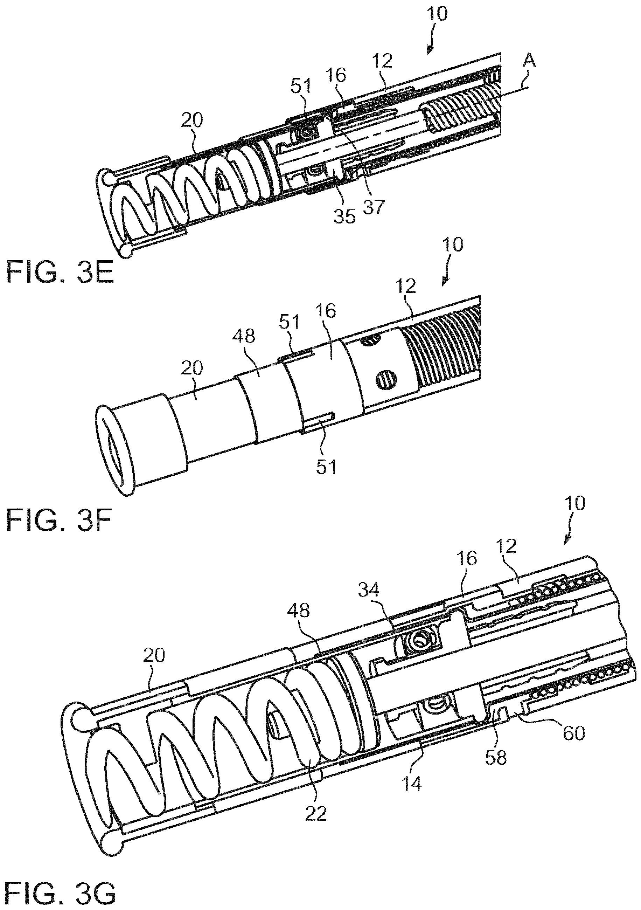

As illustrated in **Figure 3F****,** which will be further described below, the anode 16 may comprise at least two recesses 51, preferably three, distributed distinctly from one another around the circumference of the annular electrode 16. This eliminates the need to crimp the entire circumference of the anode 16, thereby preserving the underlying electrically insulating sheath 48, in particular the Teflon coating.

**Figure 2** illustrates a second embodiment for realizing the mechanical connection between the sleeve 20 and the lead body 12.

The elements with the same numerical references used for the description in Figure 1 refer to the same elements and will not be described again in detail.

In the second embodiment, unlike the first embodiment, the anode 16 is not plastically deformed.

According to the second embodiment of the invention as illustrated in **Figure 2****,** the lead 10 further comprises a retaining ring 50. The retaining ring 50 is welded to the annular electrode 16. In particular, the retaining ring 50 is welded to the inner wall 52 of the annular electrode 16. The retaining ring 50 comprises at its distal end 54 a shoulder 56.

According to the second embodiment, the retaining ring 50 is arranged, in a radial direction R of the lead, partially between the annular electrode 16 and the sleeve 20.As shown in Figure 2, the shoulder 56 abuts the distal end 14 of the lead body 12 in a direction parallel to the axis A.

The use of a retaining ring 50 provides an alternative to the first embodiment for securing the sleeve 20 relative to the lead body 12.

In both the first embodiment (shown in Figure 1) and the second embodiment (shown in Figure 2), electrically insulating adhesive 58, in particular silicone adhesive, may be introduced between the distal end 14 of the lead body 12 and the proximal end 34 of the sleeve 20 accommodated in the distal end 14 of the lead body 12 (i.e., the portion of the sleeve 20 covered by the electrically insulating sheath 48). This electrically insulating adhesive 58 may be introduced via a through-hole 60 provided in the lead body 12 at the annular electrode 16 (see Figure 1).

Thus, electrical isolation between cathode 22 and anode 16 can be further improved, as the adhesive 58 provides an additional sealing barrier.

In addition, the introduction of adhesive 58 allows minimizing any residual relative movement between the sleeve 20 and the distal end 14 of the lead body 12.

The electrically insulating adhesive 58 provides an adhesive seal 58 that ensures an axial locking by compensating the assembly clearances. In addition, the adhesive joint 58 provides a flexible stop between the proximal end 55 (opposite the distal end 54 along the axis A) of the retaining ring 50 and the shoulder 37 to prevent a progressive piercing of the insulating sleeve 48.

As in the first embodiment, the sealing means 74 is provided in the sleeve 20 to at least partially seal the lead 10 from the distal end 28.

A method for assembling a stimulation lead 10 according to the first embodiment is described below with reference to **Figures 3A-3G****.**

The elements with the same numerical references used for the description in Figure 1 refer to the same elements and will not be described again in detail.

**Figure 3A** shows a cross-sectional view of the sleeve 20 in which the retractable fixation screw 22 is housed. The electrically insulating sleeve 48 is positioned around the sleeve 20 so as to at least partially cover the retractable fixation screw 22, the electrically conductive drive shaft 24, the mouthpiece 35 and the shoulder 37, and the coiled electrical conductors 26.

**Figure 3B** depicts a next step in the method in which the electrically insulating sheath 48 is heat shrunk to the sleeve 20. The electrically insulating sleeve 48 is thereby deformed to conform to the shape of the outer circumference of the sleeve 20.

**Figure 3C** depicts a next step of the method. The proximal end 34 of the sleeve 20, opposite to the distal end 28 along the axis A, is provided with the electrically insulating thermoformed sheath 48 and is accommodated in the lead body 12 by the distal end 14 of the lead body 12 along an insertion direction D2 parallel to the longitudinal axis A of the lead 10. As described with respect to Figure 1, the distal end 14 of the lead body 12 comprises the annular electrode 16.

**Figure 3D** depicts a next step in the method in which the sleeve 20 has been partially accommodated along the direction of insertion D2 into the lead body 12 until the shoulder 37 of the mouthpiece 35 abuts an inner wall 17 of the annular electrode 16. Electrical insulation between the annular electrode 16 and the retractable fixation screw 22 is provided by the electrically insulating thermoformed sheath 48.

**Figure 3E** depicts a cross-sectional view of a subsequent method step in which the annular electrode 16 is plastically deformed by clamping or crimping to form elongated recesses 50 that extend in a direction parallel to the axis A of the lead 10.

**Figure 3F** illustrates the lead 10 in a three-dimensional schematic view. Two recesses 51 are visible around the circumference of the annular electrode 16 in Figure 3F.

As explained with reference to Figure 1, each recess 51 abuts against shoulder 37 of the mouthpiece 35 of the sleeve 20, i.e., shoulder 37 provides an axial stop for the anode 16, which mechanically holds the sleeve 20 to the lead body 12.

**Figure 3G****,** which corresponds to Figure 1, depicts a subsequent method step in which electrically insulating adhesive 58, in particular silicone adhesive, is introduced through the through-hole 60 of the ring electrode 16.

This insulating adhesive 58 disposed between the distal end 14 of the lead body 12 and the proximal end 34 of the sleeve 20 accommodated in the distal end 14 of the lead body 12 (i.e., the portion of the sleeve 20 covered by the electrically insulating sheath 48) improves the electrical isolation between the cathode 22 and the anode 16 by providing an additional sealing barrier.

In addition, the introduction of adhesive 58 allows minimizing any residual relative movement between the sleeve 20 and the distal end 14 of the lead body 12.

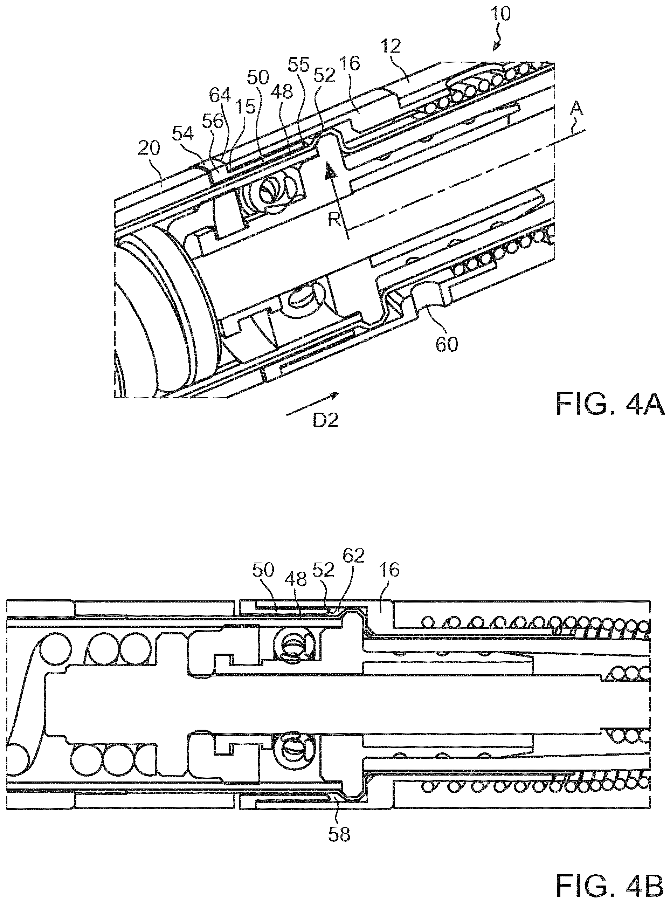

Steps of a method for assembling a stimulation lead 10 according to the second embodiment are described below with reference to Figures 4A and 4B.

The elements with the same numerical references used for the description of Figures 1 and 2 refer to the same elements and will not be described again in detail.

**Figure 4A** shows a cross-sectional view of the sleeve 20 in which the retaining ring 50 has been welded at the interface 64, between the distal end 15 of the annular electrode 16 of the lead body 10 and the shoulder 56. The interface 64 thus extends along the radial direction R. In this step of the method shown in Figure 4A, the sleeve 20 has further been inserted into the lead body 12 along an insertion direction D2 parallel to the axis A of the lead 10. As a result, the retaining ring 50 is, along a radial direction R of the lead 60, at least partially disposed between the annular electrode 16 and the sheath 48 covering the sleeve 20. Furthermore, the shoulder 56 at the distal end 54 of the retaining ring 50 is disposed, along the longitudinal direction A of the lead, between the annular electrode 16 and the sleeve 20.

**Figure 4B****,** which corresponds to Figure 2, depicts a subsequent method step in which electrically insulating adhesive 58, in particular silicone adhesive, is introduced through the through-hole 60 (only visible in Figure 4A) of the ring electrode 16.

This insulating adhesive 58 is thus disposed in the residual space 62 between the inner wall 52 of the annular electrode 16, the retaining ring 50 and the electrically insulating thermoformed sheath 48.

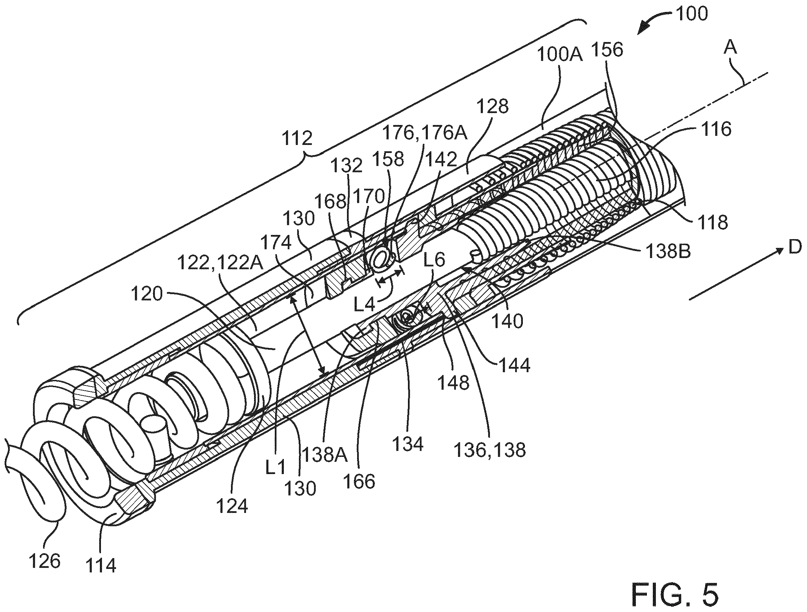

In the following, the avoidance of chatter noise between the retractable fixation screw 22, i.e. the helix electrode 22, and the sleeve 20, which constitutes a metallic distal housing portion of the lead, is explained with respect to the description of Figures 5 to 11.

**Figure 5** illustrates schematically an implantable pacing and/or defibrillation lead 100 according to a third embodiment of the present invention. In the following, the implantable pacing and/or defibrillation lead 100 is also referred as "the lead 100".

In particular, Figure 5 shows a cross-sectional view parallel to a direction of extension D of the lead 100 of a portion 112 of the lead 100. The direction of extension D of the lead 100 is parallel to a central longitudinal axis A of the lead 100.

As in the first and the second embodiments, the lead 100 is provided with an elongated lead body 100A extending along the central longitudinal axis A from a proximal end (not represented in Figure 5) to a distal end 114.

The proximal end (not represented in Figure 5) of the lead 100 is configured to be connected to an implantable medical device (not represented in Figure 5) in a manner known per se.

At least one coil conductor 116 extends from the proximal end of the lead 100 (said proximal end is not visible in the partial view of the lead 100 represented in Figure 5), through a lumen 118 of the lead 100, toward the distal end 114 of the lead 100. The coil conductor 116 is mechanically and electrically connected to a conductive driver shaft 120 moveably arranged along the central longitudinal axis A in a distal housing portion 122 of the lead 100. Although it is not visible in the view of Figure 5, the coil conductor 116 is further mechanically and electrically connected to an implantable medical device at the proximal end of the lead 100.

It is noted that the distal housing portion 122 is a distinct piece from the elongated lead body 100A.

As in the first and the second embodiments, a distal end 124 of the conductive driver shaft 120 is provided with an active fixation electrode 126, in particular a helix electrode 126. The active fixation electrode 126 is mechanically and electrically coupled to the conductive driver shaft 120 so as to form a retractable and extendable mechanism. Hence, the active fixation electrode 126 is movable along the central longitudinal axis A from a retracted position in which the active fixation electrode 126 is completely housed inside the distal housing portion 122, to an extended position, as shown in Figure 5, in which the active fixation electrode 126 extends in a distal direction at least partially beyond the distal end 114 of the distal housing portion 122 of the lead 100. The active fixation electrode 126 is electrically connected to the coil conductor 116 by means of the conductive driver shaft 120.

As the sleeve 20 in the first and the second embodiments, in the third embodiment the distal housing portion 122 of the lead 100 is provided with a cylindrical metallic body 122A of diameter L1. The use of metal, instead of plastic material, for the distal housing portion 122 allows reducing the dimension of the lead 100, in particular the longitudinal dimension of the lead 100 as explained above in reference to Figures 1 to 4B. Indeed, it allows advantageously radially superposing a ring electrode 128 provided around the elongated lead body 100A to the retractable and extendable mechanism formed by the conductive driver shaft 120 and the active fixation electrode 126 at the distal housing portion 122.

The distal housing portion 122 is partially covered by an outer insulated sheath 130 preferably made of silicone rubber or polyurethane.

As in the second embodiment, and in contrast with the first embodiment, the ring electrode 128 according to the third embodiment, i.e. the anode of the lead 100, is not plastically deformed, e.g. by crimping.

Moreover, as in the second embodiment, a retaining ring 132 is provided between the outer insulated sheath 130 and the ring electrode 128. However, the retaining ring 132 according to the third embodiments has a different shape and arrangement than the retaining ring 150 of the second embodiment, in that a portion of the retaining ring 132 extends under the outer insulated sheath 130.

An electrically insulated sheath 134, in particular a heat-shrinkable sleeve 134, more in particular a Teflon sheath 134, is provided between the distal housing portion 122, the outer insulated sheath 130, the retaining ring 132 and the ring electrode 128 for electrically isolating the ring electrode 128 from the active fixation electrode 126.

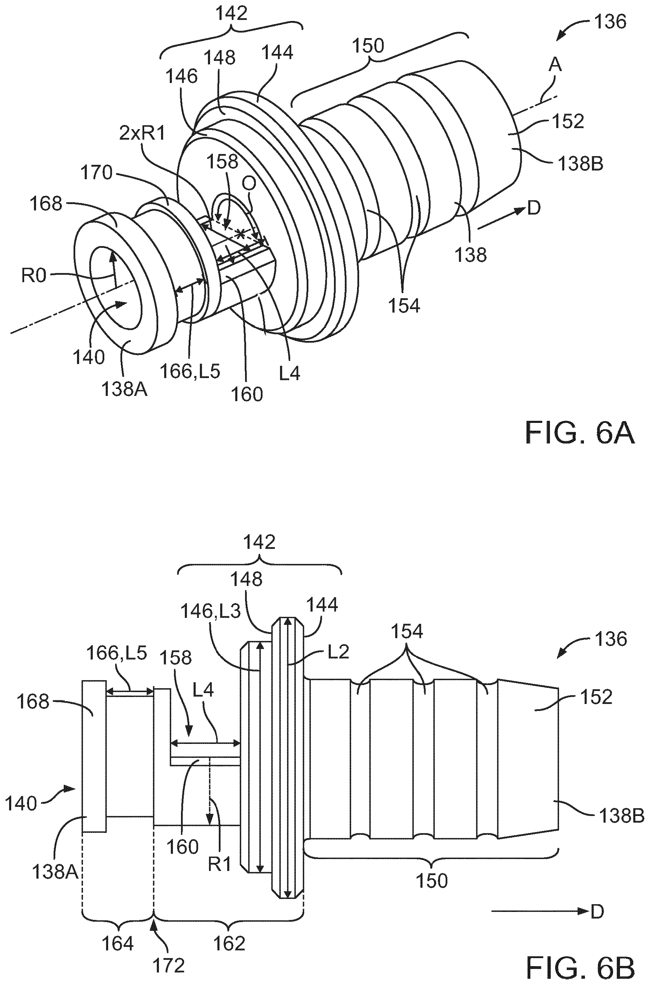

The lead 100 further comprises a guiding member 136 arranged inside the distal housing portion 122. Views of the guiding member 136 are further shown in Figures 6A and 6B to which reference is made in the following.

It is noted that the guiding member 136 is represented in Figures 1 to 4B by the mouthpiece 35. In fact, the mouthpiece 35 according to the first and second embodiments is the same as the guiding member 136 according to the third embodiment. Therefore, the description thereafter of the guiding member 136 also applies to the mouthpiece 35 according to the first and second embodiments.

The guiding member 136 comprises a tubular body 138 wherein a central lumen 140 of radius R0 extends therethrough from a distal end 138A to a proximal end 138B of the tubular body 138. As shown in Figure 5, the conductive driver shaft 120 is slideably arranged through the lumen 140 of the tubular body 138.

The tubular body 138 is electrically connected with the distal housing portion 122, in particular by means of an annular shoulder member 142 comprising a first annular shoulder 144 of diameter L2 and a second annular shoulder 146 of diameter L3, as indicated in Figure 6B. The first annular shoulder 144 and the second annular shoulder 146 extend radially from the tubular body 138. The diameter L2 is greater than the diameter L3. As a result, a step 148 is formed between the first annular shoulder 144 and the second annular shoulder 146. Moreover, the diameter L3 is substantially equal to the diameter L1 of the distal housing portion 122. As shown in Figure 5, the tubular body 138 is thus adapted for receiving the distal housing portion 122 at the step 148 of the annular shoulder member 142. The distal housing 122 thus abuts, along the central longitudinal axis A, on the first annular shoulder 144, as shown in Figure 5. The tubular body 138 can be welded to the distal housing portion 122, in particular at the step 148, preferentially by laser welding. The laser weld allows providing long term mechanical strength and electrical continuity between the tubular body 138 and the distal housing portion 122.

Between the annular shoulder member 142 and the proximal end 138B, the tubular body 138 comprises an elongated guiding portion 150. As shown in Figure 5, the central lumen 140 at the elongated guiding portion 150 is adapted for partially receiving the conductive driver shaft 120 and the coil conductor 116. The outer wall 152 of the elongated guiding portion 150 is provided with a plurality of annular grooves 154 (visible in Figures 6A and 6B) for realizing a frictional fitting with a seal member 156 of the lead 100, as shown in Figure 5. Hence, a mechanical connection is provided between the seal member 156 and the elongated guiding portion 150.

As best shown in Figures 6A and 6B, between the annular shoulder member 142 and the distal end 138A, the tubular body 138 comprises a radial opening 158 in communication with the lumen 140. In the example of Figure 6A, the radial opening 158 has an opening angle O of approximatively 180° in a cross section of the tubular body 138 perpendicular to a direction of extension D of the lead 100, which is indicated by the arrow D in Figures 6A and 6B. In a variant, the opening angle O can be comprised between 100° and 250°, in particular between 150° and 210°. The radial opening 158 has a width L4 along the direction of extension D of the lead 100.

As shown in Figures 6A and 6B, the tubular body 138 has at least one chamfered edge 160 of length T1 at the radial opening 138. The chamfered edge 160 extends along the direction of extension D of the lead 100.

As illustrated in Figure 6B, the radial opening 158 and the annular shoulder member 142 define a first portion 162 of the tubular body 138. In the first portion 162, at the radial opening 158, the tubular body 138 has an outer radius R1, as indicated in Figures 6A and 6B. As it can be understood from Figure 6A, the outer radius R1 corresponds to the sum of the radius R0 of the lumen 140 and a lateral wall thickness of the tubular body 138. It is noted that in Figure 6A, the arrow indicated by "2xR1" shows the diameter of the tubular body 138, which corresponds to two twice the radius R1.

The tubular body 138 comprises a second portion 164 extending from the distal end 138A of the tubular body 138 to the first portion 162. The second portion 164 is provided with a groove 166 of width L5 along the direction of extension D of the lead 100. The second portion 164 is provided with an external radially protruding shoulder 168 at the distal end 138A. An annular shoulder 170 is formed at the junction 172 between the first portion 162 and the second portion 164.

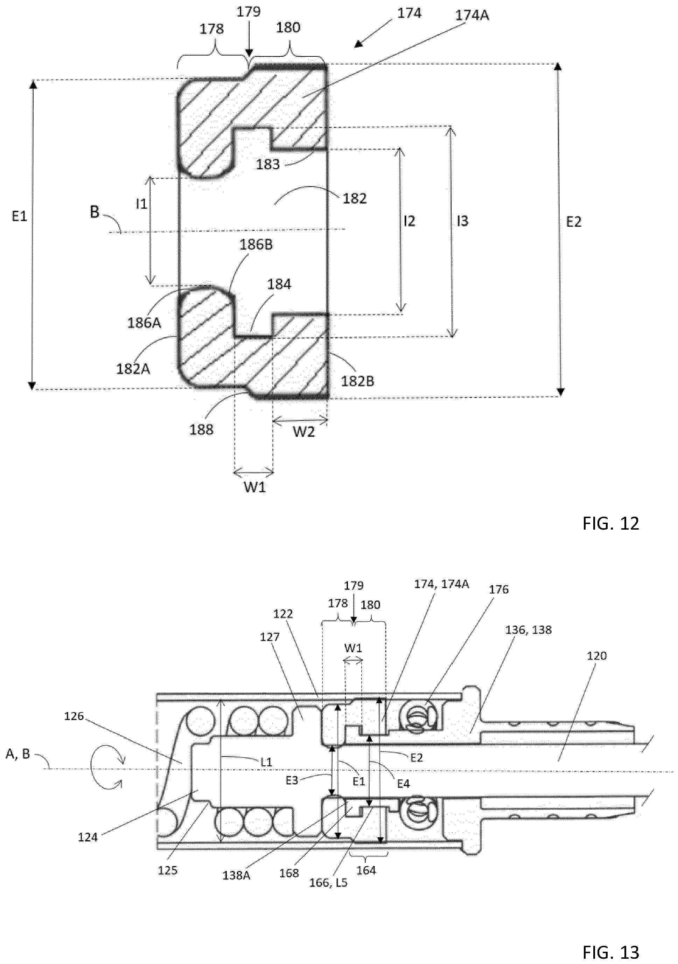

As shown in Figure 5, a sealing means 174 is provided at the second portion 164 to seal the first portion 162 of the tubular body 138 from the distal end 114 of the lead 100. The sealing means 174 is form-fitted to the second portion 162, in particular by means of the groove 166 delimited between the external radially protruding shoulder 168 and the annular shoulder 170. The sealing means 174 realizes a frictional fitting with the conductive driver shaft 120 and the distal housing portion 122, as can be seen in the cross-sectional view of Figure 5. The sealing means 174 is further described in reference to Figures 12 and 13.

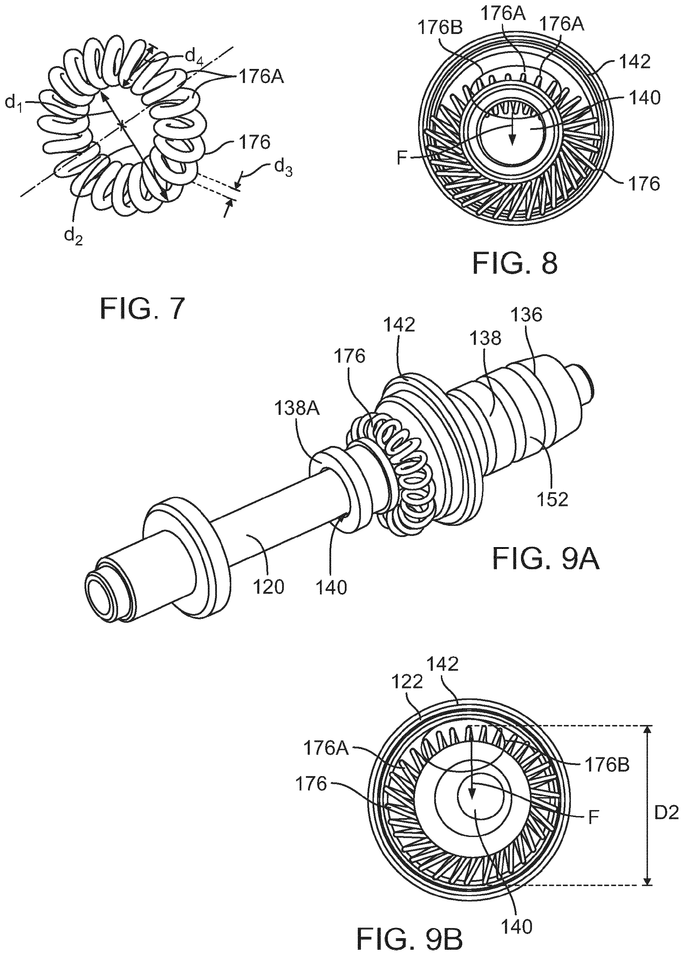

The lead 100 further comprises an electrically conductive compression spring 176. The spring 176 is preferentially a closed monowire spring 176, as illustrated in **Figure 7****.** The spring 176 is thus a closed helical compression spring 176 comprising a plurality of coils 176A along its length.

In a variant, the spring 176 is an open spring comprising two free-ends, such as an open helical compression spring. In particular, the spring 176 can be a mono-wire (i.e. a single wire) open spring.

The spring 176, when it is not arranged within the lead 100 as illustrated in Figure 7, has the following dimensions are indicated in the Table 1 below. It is understood that at the rest state of the spring 176, the spring 176 is not assembled or mounted to any elements of the lead 100. Figure 7 represents the spring 176 at its rest state.

**Table 1.**

| **Feature of the spring 176** | **Dimension of the spring 176 at its rest state** |
|---|---|
| Inner radius d1 | 0,4 mm to 1 mm |
| Outer radius d2 | 1,5 mm to 4 mm, in particular 1,5mm to 2,5 mm |
| Mono-wire thickness d3 | 0,1 to 0,5mm |
| Coil's diameter d4 | 1 to 2mm |

The inner radius d1 of the spring 176 at its rest state is less than the outer radius R1 of the first portion 162 of the tubular body 138 at the radial opening 158.

The spring 176 is preferentially made of platinum iridium, which is a biocompatible material exhibiting satisfying resistance to corrosion adapted for the application of the implantable lead 100. It is noted that the guiding member 136 is also preferentially made of platinum iridium, in particular for avoiding galvanic corrosion.

According to the present invention, the spring 176 is partially radially arranged around the tubular body 138 and its radial opening 158, such that the spring 176 is forced by its restoring force F against the conductive driver shaft 120. As a result, the coils 176A that are partially deflected within the radial opening 158 provide multiple contact points with the conductive driver shaft 120. Thereby, a continuous electrical contact between the active fixation electrode 126 and the distal housing portion 122 is realized by means of the spring 176, which allows avoiding the occurrence of chatter noise.

As the coils 176A of the spring 176 only contact the conductive driver shaft 120 at the radial opening 158, the additional friction generation caused by the coils 176A in contact with the conductive driver shaft 120 is negligible, in particular with respect to the quality of the deployment and the motion of the active fixation electrode 126.

In the followings, a method for assembling the implantable pacing and/or defibrillation lead 100 according to the third embodiment is described.

At a first step, the spring 176, which is illustrated in Figure 7, is provided around the tubular body 138 of the lead 100 at the radial opening 158 such that a portion 176B (indicated by a circle 176B on Figure 8) of the spring 176 is partially deflected inside the lumen 140 due to its restoring force F. The restoring force F of the spring 176 corresponds to the force F, which acts to bring the spring 176, in particular the portion 176B of the spring 176, to its equilibrium position. The equilibrium position of the spring 176 is the position wherein its potential energy is minimum. As the spring 176 exerts radial forces, according to the principle of minimum potential energy for a spring, the 176B portion of the spring 176 enters, in particular is clipped to, the radial opening 158, as shown in Figure 8, to reach a lower potential energy position.

In all variants of the present invention, the dimension of the opening angle O of the radial opening 158 is adapted to provide a sufficient opening for allowing a deflection of the spring 176 to a position of lower potential energy.

It is noted that, as indicated in Figure 5, the radial opening 158 has a width L4 along the direction of extension D of the lead 100 greater than the extension L6 of the spring 176 in the direction of extension D. It allows providing sufficient space for the deflection of the spring 176 towards a position of lower potential energy in the radial opening 158.

In comparison with the lead known from the state of the art, the assembly is facilitated because the spring 176 can be simply slide and clipped to the tubular member 138 at the radial opening 158, and thus does not require to be welded. Automated manufacturing is thus advantageously rendered possible.

The presence of the chamfered edges 160 allow avoiding damaging the spring 176 at the radial opening 158.

Then, at a second step illustrated by Figures 9A and 9B, the conductive driver shaft 120 is inserted via the distal end 138A through the lumen 140 of the conductive guiding member 136 thereby pushing at least partially the spring 176 outside the lumen 140 so as to provide an electrical connection between the spring 176, in particular between the coils 176A of the portion 176B, and the conductive driver shaft 120, as best shown in the cross-sectional view of Figure 9B.

The plurality of coils 176A of the portion 176B of the spring 176 provide redundancy for the contacts to ensure a reliable connection. It is further noted that the ratio of the number of coils, or spirals, of the spring 176 with respect to the length of the wire forming the spring 176 is selected so as to provide sufficient deflection to the portion 176B combined with a low bearing force on the friction surface of the conduction shaft 20. It therefore allows providing a limited parasitic friction torque.

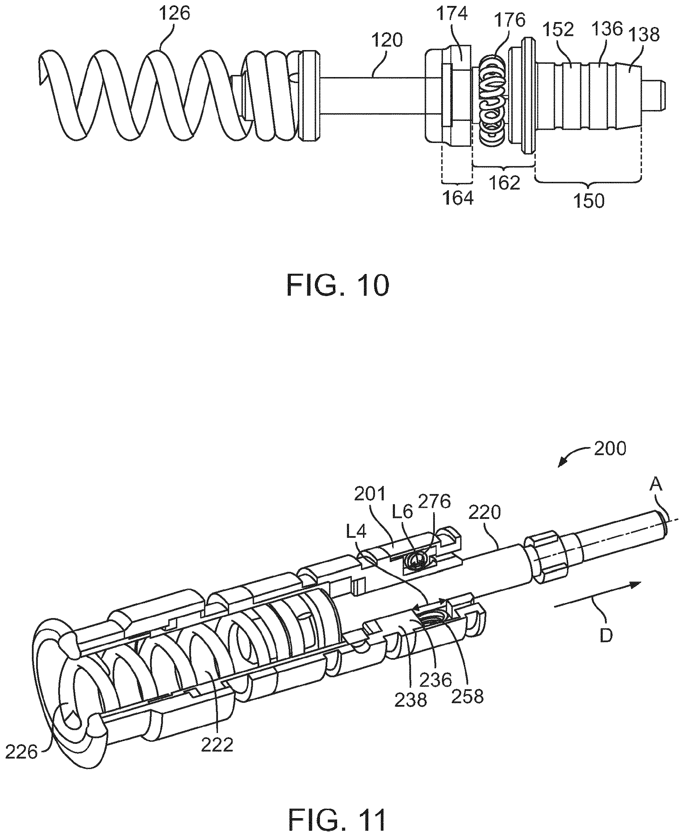

At a third step, illustrated in Figure 10, the active fixation electrode 126 is mechanically and electrically connected by laserwelding to the distal end 124 of the conductive driver shaft 120. Moreover, the sealing means 174 is provided at the second portion 164 of the tubular body 138. It is noted that the sealing means 174 is illustrated in transparency in Figure 10.

It is noted that, in an alternative, the active fixation electrode 126 can be mechanically and electrically connected by laser welding to the distal end 124 of the conductive driver shaft 120 before the insertion of the conductive driver shaft 120 in the lumen 140 of the conductive guiding member 360.

In both alternatives, an assembly comprising the active fixation electrode 126, the conductive driver shaft 120, the sealing means 174, the spring 176 and the conductive guiding member 136, as illustrated in Figure 10, is obtained.

Then, the distal housing portion 122 is provided around the assembly illustrated in Figure 10. The distal housing portion 122 is mechanically and electrically connected to the tubular member 138 of the conductive guiding member 136 by laser welding. An electrically insulated sheath 34 can be heat-shrinked (i.e. heat-retracted) at least partially around the resulting assembly, as shown in Figure 5.

The resulting assembly of the conductive driver shaft 120, the conductive guiding member 136 and the electrically conductive compression spring 176 is arranged at the distal end 114 of the lead 100 as shown in Figure 5.

**Figure 11** illustrates a fourth embodiment of an implantable pacing and/or defibrillation lead 200 according to the present invention.

Reference signs with the same tens unit than the reference signs already described and illustrated in Figures 5 to 10 will not be described in detail again but reference is made to their description above, because they relate to same functional and/or structural elements.

As explained in reference to the third embodiment, the continuous electrical contact between the active fixation electrode 226 and the distal housing portion 222 for preventing chatter noise is realized by means of the electrically conductive compression spring 276 being partially radially arranged around the tubular body 238 of the guiding member 236 and its radial opening 258, such that the electrically conductive compression spring 276 is forced by its restoring force against the conductive driver shaft 220.

In the third and the fourth embodiments, in the assembly state as illustrated in Figures 5 and 11, the radial opening 158, 258 has a width L4 along the direction of extension D of the lead 100, 200 greater than the extension L6 of the electrically conductive compression spring 176, 276 in that direction. As already explained above, it allows providing sufficient space for the deflection of the spring 176, 276 towards a position of lower potential energy in the radial opening 158, 258.

In the fourth embodiment, and in contrast with the third embodiment, the spring 276 is enclosed by an annular spring cover 201, instead of the distal housing portion 222 (as in the third embodiment).

However, in both embodiments, in the assembly state, the inner diameter L1 of the distal housing portion 122 (shown in Figure 5), or of the annular spring cover 201, is larger than the outer diameter D2 of the spring 176, 276. It is noted that the reference D2 refers to the outer diameter of the spring 176, 276 in the assembly state, as illustrated in Figure 5 and 9B, while the reference d2 refers to the outer radius of the non-assembled spring 176, 276 in its rest state, as shown in Figure 7.

The electrical connection according to the present invention is thus not provided by a radial compression of the spring 176, 276 toward the conductive driver shaft 120, 220 applied by means of a recess or cage radially compressing the spring 176, 276, as in the lead known from the state of the art.

The purpose of the enclosure provided by the distal housing portion 122 to the spring 176 in the third embodiment, or by the annular spring cover 201 to the spring 276 in the fourth embodiment, is to protect the spring 176, 276 from the external environment, in particular from damage like corrosion, or loss.

In the fourth embodiment represented in Figure 11, a sealing means 274 is provided inside the distal housing portion 222 to seal an interface between the conductive driver shaft 220 and the distal housing portion 222.

In the following, the sealing means 174 of the lead 100 according to the third embodiment is further described in reference to **Figures 12 and 13****.** The description herebelow related to the sealing means 174 also applies to the sealing means 74 of the lead 10 according to the first embodiment and the second embodiment (as shown in Figure 2).

As best shown in **Figure 12****,** the sealing means 174 comprises a sealing ring 174A of central longitudinal axis B having at least two portions 178, 180 disposed along the central longitudinal axis B of said sealing ring 174A.

The sealing means 174 is made of rubber, in particular of silicone, and is integrally formed in one-piece. The sealing means has a Shore hardness comprised between 50 to 65 Shore A.

Figure 12 illustrates a cut-view perpendicular to the central longitudinal axis B of the sealing ring 174A. The central longitudinal axis B of the sealing ring 174A corresponds to the axis of revolution of the sealing ring 174A.

The sealing ring 174A has a lumen 182 defined by an inner wall 183 of the sealing ring 174A. The lumen 182 extends along the central longitudinal axis B from a distal end 182A to a proximal end 182B of the sealing ring 174A.

It is noted that hereafter the dimensions of the sealing ring 174A are described in reference to Figure 12, which represents the sealing means 174 at rest, i.e. in an undeformed state wherein no compressive force are applied to the sealing ring 174A.

In the following, the expression "internal diameter" is to be understood as an inner diameter, i.e. a diameter of the lumen 182 defined by the inner wall 183.

The first portion 178 of the sealing ring 174A has an internal diameter 11. The second portion 180 of the sealing ring 174A has an internal diameter I2.

The internal diameter I1 of the first portion 178 of the sealing ring 174A is less than the internal diameter I2 of the second portion 180 of the sealing ring 174A.

The lumen 182 of the sealing ring 174A is provided with an internal radial recess 184 of width W1 (along the central longitudinal axis B) at a junction 179 between the first portion 178 and the second portion 180 of the sealing ring 174A. At the junction 179, the internal radial recess 184 has an internal diameter 13.

The internal diameter I2 of the second portion 180 of the sealing ring 174A is less than the internal diameter I3 of the internal radial recess 184 of the sealing ring 174A.

In the following, the expression "external diameter" is to be understood as an outer diameter of the sealing ring 174A.

The first portion 178 of the sealing ring 174A has an external diameter E1. The second portion 180 of the sealing ring 174A has an external diameter E2.

The external diameter E1 of the first portion 178 of the sealing ring 174A is less than the external diameter E2 of the second portion 180 of the sealing ring 174A. Hence, as shown in Figure 12, the first portion 178 and the second portion 180 are connected by a slope 188 at the junction 179.

The inner wall 183 at the first portion 178 is provided with a rounded edge 186A at the distal end 182A. The inner wall 183 at the first portion 178 is further provided with a rounded edge 186B between the first portion 178 and the junction 179.

The second portion 180 has a width W2 along the central longitudinal axis B.

**Figure 13** illustrates a cut-view perpendicular to the central longitudinal axis A of the lead 100. In particular, Figure 13 represents a partial view of Figure 5. Hence, the elements with the same numerical references used for the description in Figure 5 refer to the same elements and will not be described again in detail.

The conductive driver shaft 120 comprises a shoulder 125 at its distal end 124. The conductive driver shaft 120 further comprises an external radially protruding shoulder 127. The external radially protruding shoulder 127 is longitudinally arranged between the active fixation electrode 126 and the sealing means 174.

As already mentioned above with respect to Figures 6A and 6B, the second portion 164 of the tubular body 138 of the guiding member 136 is provided with the groove 166 of width L5 along the central longitudinal axis A of the lead 100. The width L5 of the groove 166 is substantially equal or greater than the width W2 of the second portion 180 of the sealing ring 174A. Hence, the second portion 180 of the sealing ring 174A can be received in the groove 166. In particular, the second portion 180 of the sealing ring 174A can be maintained to the groove 166 of the guiding member 136 by a form-fit connection and/or a friction-fit connection.

The second portion 164 of the tubular body 138 of the guiding member 136 is further provided with the external radially protruding shoulder 168 at the distal end 138A of the tubular body 138. The external radially protruding shoulder 168 is form-fitted into the internal radial recess 184 of width W1 of the sealing ring 174A.

As shown in **Figure 13****,** the sealing means 174 is arranged around the conductive driver shaft 120 and the tubular body 138 for sealing the distal housing portion 122 from the distal end of the lead.

Indeed, the first portion 178 of the sealing ring 174A is arranged around, in particular directly arranged around (i.e. in surface contact with), the conductive driver shaft 120. More precisely, the first portion 178 is longitudinally arranged between the external radially protruding shoulder 127 of conductive driver shaft 120 and the external radially protruding shoulder 168 at the distal end 138A of the tubular body 138.

The section of the conductive driver shaft 120 passing through the lumen 182 of the sealing ring 174A at the first portion 178 of the sealing ring 174A has an external diameter E3.

The second portion 180 of the sealing ring 174B is arranged around, in particular directly arranged around (i.e. in surface contact with) the second portion 164 of the tubular body 138 of the guiding member 136.

At the external radially protruding shoulder 168, the tubular body 138 has an external diameter E4.

The end portion housing 122, wherein at least the second portion 164 of the tubular body 138 and the conductive driver shaft 120 are received, has the internal diameter L1.

The first portion 178 of the sealing ring 174A has an external diameter E1 strictly less than the internal diameter L1 of the distal housing portion 122.

In particular, the percentage difference of diameter between the external diameter E1 of the first portion 178 of the sealing ring 174A and the internal diameter L1 of the distal housing portion 22 is comprised between 3 % and 30%, in particular between 5% to 20%
The first portion 178 of the sealing ring 174A has an internal diameter I1 strictly less than the external diameter E3 of the conductive driver shaft 120.

In particular, the percentage difference between the internal diameter I1 of the first portion 178 of the sealing ring 174A and an external diameter E3 of the conductive driver shaft 120 is comprised between 0,05 % and 20%, in particular between 1% and 10%.

The above-mentioned range of percentage difference allows the conductive driver shaft 120 to rotate and slide with respect to the sealing ring 174A along the longitudinal axis A while preventing fluid communication at the interface between the conductive driver shaft 120 and the first portion 178 of the sealing ring 174A.

The second portion 180 of the sealing ring 174A has an external diameter E2 strictly more than the internal diameter L1 of the distal housing portion 122.

In particular, the percentage difference between the external diameter E2 of the second portion 180 of the sealing ring 174A and the internal diameter L1 of the distal housing portion 122 is comprised between 3 % and 30%, in particular between 5% and 20%.

Hence, the dimensions of the sealing ring 174A of the sealing means 174 allows avoiding fluidic communication from the distal end of the lead wherein blood can enter the distal housing portion 122. It thus allows sealing the distal housing portion 122, in particular wherein the electrically conductive spring 176 is housed for providing a continuous electrical connection. The sealing means 174 thus decrease the risk of surface and/or volume corrosion inside the distal housing portion 122, in particular of the electrically conductive spring 176.

The sealing means 174 is thus designed such that its first portion 178 comprises one constrain zone at the interface between the inner wall 183 and the conductive driver shaft 120 whereas its second portion 180 comprises two constrain zones, respectively: at the interface between the inner wall 183 and the tubular body 138, and, at the interface between the second portion 180 and the distal housing portion 122.

Hence, in contrast with the second portion 180 of the sealing means 174, there is no, or at least less, compressive forces exerted on the first portion 178 by the distal housing portion 122. This is because there is no direct surface contact between the external wall of the first portion 178 of the sealing means 174 and the internal wall of the distal housing portion 122. Thereby, it allows reducing the friction applied by the sealing means 174 on the conductive driver shaft 120. Indeed, the first portion 178, in contrast with the second portion 180, is not squeezed (i.e. compressed or elastically deformed) and maintained between two opposite surfaces. In fact, the second portion 180 of the sealing means 174 is compressed against the internal surface of the distal housing portion 122 and the external surface of the tubular body 138.

In view of the above, the conductive driver shaft 120 can thus smoothly rotate and translate, even within the first portion 178, so as to ensure a smooth deployment of the active fixation electrode without unwanted jerky motion of the conductive driver shaft 120 caused by friction.

It is noted that the internal diameter E3 of the first portion 178 of the sealing means 174 is configured to provide sealing properties at the interface between the inner wall 183 and the conductive driver shaft 120. Hence, the sealing properties at the interface between the inner wall 183 of the sealing means 174 and the conductive driver shaft 120 are ensured, despite any displacement of the conductive driver shaft 120 within the first portion 178 of the sealing means 174.

Because the second portion 180 of the sealing means 174 is radially compressed between the internal surface of the distal housing portion 122 and the external surface of the tubular body 138, the second portion 180 is maintained by form-fit and friction-fit connections between the distal housing portion 122 and the tubular body 138. It allows holding the position of the sealing means while providing sufficient sealing properties
It is noted that the Shore hardness of the sealing means 174 and the ratio of diameter differences can be advantageously selected in combination so as to provide appropriate sealing properties with minimum friction on the conductive driver shaft 120.

It is further noted that the structural features of the sealing means 174 are not intrinsically linked to the presence or not of a radial opening 158 provided at the first portion 162 of the tubular body 138 of the guiding member 136.

Although the embodiments have been described in relation to particular examples, the invention is not limited and numerous alterations to the disclosed embodiments can be made without departing from the scope of this invention which is defined by the appended claims.

### Reference list

10: lead according to the first and the second embodiments
100: lead according to the third embodiment
100A: elongated lead body
112: portion of the lead 100
114: distal end of the lead
26, 116: coil conductor
118: lumen
24, 120, 220: conductive driver shaft
122, 222: distal housing portion
122A: cylindrical metallic body of the distal housing portion 122
124: distal end of the conductive driver shaft
125; shoulder of the conductive driver shaft
22, 126, 226: active fixation electrode
127: external radially protruding shoulder of the conductive driver shaft
128: ring electrode
130: outer insulated sheath
132: retaining ring
134: electrically insulated sheath
35, 136, 236: guiding member
138, 238: tubular body of the guiding member
138A: distal end
138B: proximal end
140: central lumen of the tubular body
142: annular shoulder member
144: first annular shoulder
146: second annular shoulder
148: step
150: elongated guiding portion
152: outer wall
154: annular grooves
156: seal member
158, 258: radial opening
160: chamfered edge
162: first portion
164: second portion
166: groove
168: external radially protruding shoulder
170: annular shoulder
172: junction between the first portion 162 and the second portion 164
74, 174: sealing means
174: sealing ring
176, 276: electrically conductive compression spring
176A: coil
176B: portion
178: first portion of the sealing ring
179: junction
180: second portion of the sealing ring
182: lumen of the sealing ring
183: inner wall of the sealing ring
182A: distal end of the sealing ring
182B: proximal end of the sealing ring
184: internal radial recess of the sealing ring
186A. 186B: rounded edge
188: slope
200: lead according to the fourth embodiment
201: annular spring cover
A: central longitudinal axis of the lead 100, 200
B: central longitudinal axis of sealing ring 174A
d1: inner radius of the spring 176 at rest
d2: outer radius of the spring 176 at rest
d3: monowire thickness
d4: coil's diameter
D: direction of extension of the lead 100, 200
D2 (in the first and second embodiments): direction of insertion
D2 (in the third and fourth embodiments): outer diameter of the springs 176, 276 in the assembled state
E1, E2, E3, E4: external diameter
F: restoring force
I1, I2, I3: internal diameter of the sealing ring 174A
L1 (in the first and second embodiments): distance between the cathode 22 and the anode 16
L1 (in the third and fourth embodiments): diameter of the distal housing portion 122 or of the annular spring cover 201
L2: diameter of first annular shoulder 144
L3: diameter of second annular shoulder
L4: width of the radial opening 158, 258
L5: width of the groove 166
L6: extension of the spring the direction of extension D
O: opening angle
R0: inner radius of the lumen 140
R1: outer radius of tubular body 138
T1: chamfered edge
W1, W2: width of the sealing ring 174A

## Claims

1. An implantable pacing and/or defibrillation lead, said lead (10, 100, 200) extending from a proximal end to a distal end (114), wherein the proximal end of the lead (10, 100, 200) is configured to be connected to an implantable medical device, the lead (10, 100, 200) comprising:
- an active fixation electrode (22, 126, 226) at the distal end (114) of the lead (10, 100, 200), which is mechanically and electrically coupled to a conductive driver shaft (24, 120, 220) moveably arranged in a distal housing portion (122, 222) of the lead (10, 100, 200),
wherein the active fixation electrode (22, 126, 226) is movable from a retracted position in which the active fixation electrode (22, 126, 226) is completely housed inside the distal housing portion (122, 222), to an extended position, in which the active fixation electrode (126, 226) extends in a distal direction at least partially beyond the distal housing portion (122, 222), and
- an electrically conductive compression spring (176, 276),
said lead (10, 100, 200) further comprising:
- a guiding member (35, 136, 236) arranged inside the distal housing portion (122, 222) and comprising a tubular body (138, 238) electrically connected with the distal housing portion (122, 222), wherein
the conductive driver shaft (24, 120, 220) is slideably arranged through a lumen (140) extending through the tubular body (138, 238),
wherein the tubular body (138, 238) further comprises a radial opening (158, 258) in communication with the lumen (140), and
wherein the electrically conductive compression spring (176, 276) is partially radially arranged around the tubular body (138, 238) and its radial opening (158, 258), such that the electrically conductive compression spring (176, 276) is forced by its restoring force against the conductive driver shaft (24, 120, 220).

2. An implantable pacing and/or defibrillation lead according to claim 1, wherein the electrically conductive compression spring (176, 276) is a closed coiled helical spring (176, 276).

3. An implantable pacing and/or defibrillation lead according to claim 1, wherein the electrically conductive compression spring (176, 276) is an open coiled helical spring (176, 276) comprising two free-ends.

4. An implantable pacing and/or defibrillation lead according to claim 1 or 2, wherein at least a portion (162) of the tubular body (138) provided with the radial opening (158) has an outer radius (R1) greater than an inner radius (d1) of the electrically conductive compression spring (716) at its rest state.

5. An implantable pacing and/or defibrillation lead according one of the preceding claims, wherein the radial opening (158, 258) has an opening angle (O) comprised between 100° and 250°, in particular between 150° and 210°, in a cross section of the tubular body (138, 238) perpendicular to a direction of extension (D) of the lead (100, 200).

6. An implantable pacing and/or defibrillation lead according to one of the preceding claims, wherein the radial opening (158, 258) has a width (L4) along a direction of extension (D) of the lead (100, 200) greater than the extension (L6) of the electrically conductive compression spring (158, 258) in that direction (D).

7. An implantable pacing and/or defibrillation lead according to one of the preceding claims, wherein the inner diameter (L1) of the distal housing portion (122) is larger than the diameter (D2) of the electrically conductive compression spring (158) at least partially radially arranged around the tubular body (138, 238) and its radial opening (158, 258).

8. An implantable pacing and/or defibrillation lead according to one of the preceding claims, wherein the tubular body (138, 238) has at least one chamfered edge (160) at the radial opening (158, 258).

9. An implantable pacing and/or defibrillation lead according to one of the preceding claims, wherein the tubular body (138) comprises at least:
- a first portion (162) provided with the radial opening (158) and a member (37, 142, 144) of the tubular body (138) contacting the distal housing portion (122), and
- a second portion (164) extending from a distal end (138A) of the tubular body (138) to the first portion (162),
and wherein a sealing means (174) is provided at the second portion (164) to seal the first portion (162) of the tubular body (138) from the distal end (138A) of the lead (100).

10. An implantable pacing and/or defibrillation lead according to one of the preceding claims, wherein the tubular body (138) of the guiding member (136) is mechanically and electrically connected to the distal housing portion (22) by means of a welding.

11. Method for assembling an implantable pacing and/or defibrillation lead (10, 100, 200) according to one of claims 1 to 10, comprising the steps of:
a) providing the electrically conductive compression spring (176, 276) around the tubular body (138, 238) of the guiding member (136, 236) at the radial opening (158, 258) such that a portion (176B) of the electrically conductive compression spring (176, 276) is partially deflected inside the lumen (140) due to its restoring force (F), then
b) inserting the conductive driver shaft (120, 220) through the lumen (140) of the conductive guiding member (136, 236) thereby pushing partially the compression spring (176, 276) outside the lumen (140) so as to provide an electrical connection between the electrically conductive compression spring (176, 276) and the conductive driver shaft (120, 220), and then
c) mechanically and electrically connecting the tubular body (138, 238) of the guiding member (136, 236) to the distal housing portion (122, 222) of the lead (100, 200).

12. Method according to claim 11, wherein step b) further comprises providing a sealing means (174) at the distal end (138A, 162) of the tubular body (138) to seal it from the distal end (114) of the lead (100).

13. Method according to claim 12, wherein step c) comprises welding the tubular body (138, 238) to the distal housing portion (122, 222) of the lead (100, 200), in particular by laser welding.

14. An implantable pacing and/or defibrillation lead, said lead (10, 100) extending from a proximal end to a distal end (114), wherein the proximal end of the lead (10, 100) is configured to be connected to an implantable medical device, the lead (10, 100) comprising:
- an active fixation electrode (22, 126) at the distal end (114) of the lead (10, 100), which is mechanically and electrically coupled to a conductive driver shaft (24, 120) moveably arranged in a distal housing portion (122) of the lead,
wherein the active fixation electrode (126) is movable from a retracted position in which the active fixation electrode (22, 126) is completely housed inside the distal housing portion (122), to an extended position, in which the active fixation electrode (126) extends in a distal direction at least partially beyond the distal housing portion (122),
said lead (10, 100) further comprising:
- a guiding member (35, 136) arranged inside the distal housing portion (122) and comprising a tubular body (138) electrically connected with the distal housing portion (122), wherein the conductive driver shaft (24, 120) is slideably and rotatably arranged through a lumen (140) extending through the tubular body (138), and
- a sealing means (174) for sealing the distal housing portion (122) from the distal end (138A) of the lead (100),
the sealing means (174) comprising a sealing ring (174A) having at least two portions (178, 180) with respect to a central longitudinal axis (B) of said sealing ring (174A),
a first portion (178) of the sealing ring (174A) being arranged around the conductive driver shaft (24, 120) and a second portion (180) of the sealing ring (174A) being arranged around the tubular body (138),
the first portion (178) of the sealing ring (174A) having an external diameter (E1) strictly less than an internal diameter (L1) of the distal housing portion (122), and
the first portion (178) of the sealing ring (174A) having an internal diameter (11) strictly less than an external diameter (E3) of the conductive driver shaft (24, 120), and
the second portion (180) of the sealing ring (174A) having an external diameter (E2) strictly more than the internal diameter (L1) of the distal housing portion (122),
wherein a distal end (138A) of the tubular body (138) is provided with an external radially protruding shoulder (168), said external radially protruding shoulder (168) being form-fitted into a corresponding internal radial recess (184) of the sealing ring (174A) of the sealing means (174).

15. An implantable pacing and/or defibrillation lead according to claim 14, wherein the percentage difference of diameter between the external diameter (E1) of the first portion (178) of the sealing ring (174A) and the internal diameter (L1) of the distal housing portion (122) is comprised between 3 % and 30%, in particular between 5% to 20%.

16. An implantable pacing and/or defibrillation lead according to claim 14 or 15, wherein the percentage difference between the internal diameter (11) of the first portion (178) of the sealing ring (174A) and an external diameter (E3) of the conductive driver shaft (120) be comprised between 0,05 % and 20%, in particular between 1% to 10%.

17. An implantable pacing and/or defibrillation lead according to claim 14, 15 or 16, wherein the percentage difference between the external diameter (E2) of the second portion (180) of the sealing ring (174A) and the internal diameter (L1) of the distal housing portion (122) is comprised between 0,5 % and 20%, in particular between 1% to 10%.

18. An implantable pacing and/or defibrillation lead according to one of claims 14 to 16, wherein the internal diameter (11) of the first portion (178) of the sealing ring (174A) is less than the internal diameter (12) of the second portion (180) of the sealing ring (174A).

19. An implantable pacing and/or defibrillation lead according to claim 14, wherein the internal radial recess (184) of the sealing ring (184A) is provided at a junction (179) between the first portion (178) and the second portion (180) of the sealing ring (174A).

20. An implantable pacing and/or defibrillation lead according to one of claims 14 to 19, wherein the first portion (178) of the sealing ring (174A) has a rounded edge along its internal diameter (11).

21. An implantable pacing and/or defibrillation lead according to one of claims 14 to 20, wherein the sealing means (174) is integrally formed in one-piece.

22. An implantable pacing and/or defibrillation lead according to one of claims 14 to 21, wherein the sealing means (174) is made of rubber, in particular of silicone, and has a Shore hardness comprised between 50 to 65 Shore A.

23. An implantable pacing and/or defibrillation lead according to claim 1, further comprising:
- a sealing means (174) for sealing the distal housing portion (122) from the distal end (138A) of the lead (100),
the sealing means (174) comprising a sealing ring (174A) having at least two portions (178, 180) with respect to a central longitudinal axis (B) of said sealing ring (174A), a first portion (178) of the sealing ring (174A) being arranged around the conductive driver shaft (24, 120) and a second portion (180) of the sealing ring (174A) being arranged around the tubular body (138),
the first portion (178) of the sealing ring (174A) having an external diameter (E1) strictly less than an internal diameter (L1) of the distal housing portion (122), and
the first portion (178) of the sealing ring (174A) having an internal diameter (11) strictly less than an external diameter (E3) of the conductive driver shaft (24, 120), and
the second portion (180) of the sealing ring (174A) having an external diameter (E2) strictly more than the internal diameter (L1) of the distal housing portion (122).

## Patentansprüche

1. Eine implantierbare Schrittmacher- und/oder Defibrillationsleitung, wobei die Leitung (10, 100, 200) sich von einem proximalen Ende zu einem distalen Ende (114) erstreckt, wobei das proximale Ende der Leitung (10, 100, 200) dazu eingerichtet ist, mit einem implantierbaren medizinischen Gerät verbunden zu werden, die Leitung (10, 100, 200) umfasst:
- eine aktive Fixationselektrode (22, 126, 226) am distalen Ende (114) der Leitung (10, 100, 200), die mechanisch und elektrisch mit einer leitfähigen Antriebswelle (24, 120, 220) gekoppelt ist, beweglich in einem distalen Gehäuseteil (122, 222) der Leitung (10, 100, 200) angeordnet ist,
wobei die aktive Fixationselektrode (22, 126, 226) bewegbar ist von einer zurückgezogenen Position, in der die aktive Fixationselektrode (22, 126, 226) vollständig innerhalb des distalen Gehäuseteils (122, 222) untergebracht ist, bis in eine ausgefahrene Position, in der die aktive Fixationselektrode (126, 226) in distaler Richtung zumindest teilweise über das distale Gehäuseteil (122, 222) hinausgeht, und
- eine elektrisch leitfähige Druckfeder (176, 276),
wobei die Leitung (10, 100, 200) weiterhin umfasst:
- ein Führungselement (35, 136, 236), das innerhalb des distalen Gehäuseteils (122, 222) angeordnet ist und einen röhrenförmigen Körper (138, 238) umfasst, der elektrisch mit dem distalen Gehäuseteil (122, 222) verbunden ist, wobei
die leitfähige Antriebswelle (24, 120, 220) verschiebbar durch ein Lumen (140) angeordnet ist, das sich durch den röhrenförmigen Körper (138, 238) erstreckt,
wobei der röhrenförmige Körper (138, 238) ferner eine radiale Öffnung (158, 258) aufweist, die mit dem Lumen (140) in Verbindung steht, und
wobei die elektrisch leitfähige Druckfeder (176, 276) teilweise radial um den röhrenförmigen Körper (138, 238) und dessen radiale Öffnung (158, 258) angeordnet ist, so dass die elektrisch leitfähige Druckfeder (176, 276) durch ihre Rückstellkraft gegen die leitfähige Antriebswelle (24, 120, 220) gedrückt wird.

2. Eine implantierbare Schrittmacher- und/oder Defibrillationsleitung nach Anspruch 1, wobei die elektrisch leitfähige Druckfeder (176, 276) eine geschlossene Schraubendruckfeder (176, 276) ist.

3. Eine implantierbare Schrittmacher- und/oder Defibrillationsleitung nach Anspruch 1, wobei die elektrisch leitfähige Druckfeder (176, 276) eine offene Schraubendruckfeder (176, 276) mit zwei freien Enden ist.

4. Eine implantierbare Schrittmacher- und/oder Defibrillationsleitung nach Anspruch 1 oder 2, wobei ein Abschnitt (162) des röhrenförmigen Körpers (138), der mit der radialen Öffnung (158) versehen ist, einen äußeren Radius (R1) aufweist, der größer ist als ein innerer Radius (d1) der elektrisch leitfähigen Druckfeder (716) im Ruhezustand.

5. Eine implantierbare Schrittmacher- und/oder Defibrillationsleitung nach einem der vorhergehenden Ansprüche, wobei die radiale Öffnung (158, 258) einen Öffnungswinkel (O) zwischen 100° und 250° aufweist, insbesondere zwischen 150° und 210°, in einem Querschnitt des röhrenförmigen Körpers (138, 238), der senkrecht zur Verlängerungsrichtung (D) der Leitung (100, 200) steht.

6. Eine implantierbare Schrittmacher- und/oder Defibrillationsleitung nach einem der vorhergehenden Ansprüche, wobei die radiale Öffnung (158, 258) eine Breite (L4) entlang einer Verlängerungsrichtung (D) der Leitung (100, 200) aufweist, die größer ist als die Ausdehnung (L6) der elektrisch leitfähigen Druckfeder (158, 258) in dieser Richtung (D).

7. Eine implantierbare Schrittmacher- und/oder Defibrillationsleitung nach einem der vorhergehenden Ansprüche, wobei der Innendurchmesser (L1) des distalen Gehäuseteils (122) größer ist als der Durchmesser (D2) der elektrisch leitfähigen Druckfeder (158), die zumindest teilweise radial um den röhrenförmigen Körper (138, 238) und dessen radiale Öffnung (158, 258) angeordnet ist.

8. Eine implantierbare Schrittmacher- und/oder Defibrillationsleitung nach einem der vorhergehenden Ansprüche, wobei der röhrenförmige Körper (138, 238) mindestens eine abgeschrägte Kante (160) an der radialen Öffnung (158, 258) aufweist.

9. Eine implantierbare Schrittmacher- und/oder Defibrillationsleitung nach einem der vorhergehenden Ansprüche, wobei der röhrenförmige Körper (138) zumindest Folgendes umfasst:
- einen ersten Abschnitt (162) mit der radialen Öffnung (158) und einem Element (37, 142, 144) des röhrenförmigen Körpers (138), das das distale Gehäuseteil (122) berührt, und
- einen zweiten Abschnitt (164), der sich von einem distalen Ende (138A) des röhrenförmigen Körpers (138) bis zum ersten Abschnitt (162) erstreckt,
und wobei am zweiten Abschnitt (164) ein Dichtungsmittel (174) vorgesehen ist, um den ersten Abschnitt (162) des röhrenförmigen Körpers (138) vom distalen Ende (138A) der Leitung (100) abzudichten.

10. Eine implantierbare Schrittmacher- und/oder Defibrillationsleitung nach einem der vorhergehenden Ansprüche, wobei der röhrenförmige Körper (138) des Führungselements (136) mechanisch und elektrisch durch Schweißen mit dem distalen Gehäuseteil (22) verbunden ist.

11. Verfahren zur Montage einer implantierbaren Schrittmacher- und/oder Defibrillationsleitung (10, 100, 200) nach einem der Ansprüche 1 bis 10, umfassend die Schritte:
a) Anordnen der elektrisch leitfähigen Druckfeder (176, 276) um den röhrenförmigen Körper (138, 238) des Führungselements (136, 236) an der radialen Öffnung (158, 258), so dass ein Abschnitt (176B) der elektrisch leitfähigen Druckfeder (176, 276) aufgrund ihrer Rückstellkraft (F) teilweise in das Lumen (140) abgelenkt wird, dann
b) Einführen der leitfähigen Antriebswelle (120, 220) durch das Lumen (140) des leitfähigen Führungselements (136, 236), wodurch die Druckfeder (176, 276) teilweise aus dem Lumen (140) hinausgedrückt wird, um eine elektrische Verbindung zwischen der elektrisch leitfähigen Druckfeder (176, 276) und der leitfähigen Antriebswelle (120, 220) bereitzustellen, und dann
c) Mechanisches und elektrisches Verbinden des röhrenförmigen Körpers (138, 238) des Führungselements (136, 236) mit dem distalen Gehäuseteil (122, 222) der Leitung (100, 200).

12. Verfahren nach Anspruch 11, wobei Schritt b) weiterhin das Bereitstellen eines Dichtungsmittels (174) am distalen Ende (138A, 162) des röhrenförmigen Körpers (138) umfasst, um diesen vom distalen Ende (114) der Leitung (100) abzudichten.

13. Verfahren nach Anspruch 12, wobei Schritt c) das Verschweißen des röhrenförmigen Körpers (138, 238) mit dem distalen Gehäuseteil (122, 222) der Leitung (100, 200) umfasst, insbesondere durch Laserschweißen.

14. Eine implantierbare Schrittmacher- und/oder Defibrillationsleitung, wobei die Leitung (10, 100) sich von einem proximalen Ende zu einem distalen Ende (114) erstreckt, wobei das proximale Ende der Leitung (10, 100) dazu eingerichtet ist, mit einem implantierbaren medizinischen Gerät verbunden zu werden, die Leitung (10, 100) umfassend:
- eine aktive Fixationselektrode (22, 126) am distalen Ende (114) der Leitung (10, 100), die mechanisch und elektrisch mit einer leitfähigen Antriebswelle (24, 120) gekoppelt ist, die beweglich in einem distalen Gehäuseteil (122) der Leitung angeordnet ist,
wobei die aktive Fixationselektrode (126) bewegbar ist von einer zurückgezogenen Position, in der die aktive Fixationselektrode (22, 126) vollständig innerhalb des distalen Gehäuseteils (122) untergebracht ist, in eine ausgefahrene Position, in der die aktive Fixationselektrode (126) in distaler Richtung zumindest teilweise über das distale Gehäuseteil (122) hinausgeht,
wobei die Leitung (10, 100) weiterhin umfasst:
- ein Führungselement (35, 136), das innerhalb des distalen Gehäuseteils (122) angeordnet ist und einen röhrenförmigen Körper (138) umfasst, der elektrisch mit dem distalen Gehäuseteil (122) verbunden ist, wobei
die leitfähige Antriebswelle (24, 120) verschiebbar und drehbar durch ein Lumen (140) angeordnet ist, das sich durch den röhrenförmigen Körper (138) erstreckt, und
- ein Dichtungsmittel (174) zum Abdichten des distalen Gehäuseteils (122) vom distalen Ende (138A) der Leitung (100),
wobei das Dichtungsmittel (174) einen Dichtring (174A) umfasst, der mindestens zwei Abschnitte (178, 180) in Bezug auf eine zentrale Längsachse (B) des Dichtrings (174A) aufweist,
ein erster Abschnitt (178) des Dichtrings (174A) der um die leitfähige Antriebswelle (24, 120) angeordnet ist, und ein zweiter Abschnitt (180) des Dichtrings (174A) der um den röhrenförmigen Körper (138) angeordnet ist,
wobei der erste Abschnitt (178) des Dichtrings (174A) einen äußeren Durchmesser (E1) aufweist, der strikt kleiner ist als ein innerer Durchmesser (L1) des distalen Gehäuseteils (122),
und der erste Abschnitt (178) des Dichtrings (174A) einen inneren Durchmesser (11) aufweist, der strikt kleiner ist als ein äußerer Durchmesser (E3) der leitfähigen Antriebswelle (24, 120),
und der zweite Abschnitt (180) des Dichtrings (174A) einen äußeren Durchmesser (E2) aufweist, der strikt größer ist als der innere Durchmesser (L1) des distalen Gehäuseteils (122),
wobei ein distales Ende (138A) des röhrenförmigen Körpers (138) mit einer nach außen radial vorspringenden Schulter (168) versehen ist, die außen radial vorspringende Schulter erstreckt sich formschlüssig in eine entsprechende innere radiale Vertiefung (184) des Dichtrings (174A) des Dichtungsmittels (174).

15. Eine implantierbare Schrittmacher- und/oder Defibrillationsleitung nach Anspruch 14, wobei der prozentuale 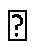 nterschied zwischen dem äußeren Durchmesser (E1) des ersten Abschnitts (178) des Dichtrings (174A) und dem inneren Durchmesser (L1) des distalen Gehäuseteils (122) zwischen 3 % und 30 % liegt, insbesondere zwischen 5 % und 20 %.

16. Eine implantierbare Schrittmacher- und/oder Defibrillationsleitung nach Anspruch 14 oder 15, wobei der prozentuale 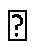 nterschied zwischen dem inneren Durchmesser (11) des ersten Abschnitts (178) des Dichtrings (174A) und einem äußeren Durchmesser (E3) der leitfähigen Antriebswelle (120) zwischen 0,05 % und 20 % liegt, insbesondere zwischen 1 % und 10 %.

17. Eine implantierbare Schrittmacher- und/oder Defibrillationsleitung nach Anspruch 14, 15 oder 16, wobei der prozentuale 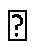 nterschied zwischen dem äußeren Durchmesser (E2) des zweiten Abschnitts (180) des Dichtrings (174A) und dem inneren Durchmesser (L1) des distalen Gehäuseteils (122) zwischen 0,5 % und 20 % liegt, insbesondere zwischen 1 % und 10 %.

18. Eine implantierbare Schrittmacher- und/oder Defibrillationsleitung nach einem der Ansprüche 14 bis 16, wobei der innere Durchmesser (11) des ersten Abschnitts (178) des Dichtrings (174A) kleiner ist als der innere Durchmesser (12) des zweiten Abschnitts (180) des Dichtrings (174A).

19. Eine implantierbare Schrittmacher- und/oder Defibrillationsleitung nach Anspruch 14, wobei die innere radiale Vertiefung (184) des Dichtrings (184A) an einer Verbindungsstelle (179) zwischen dem ersten Abschnitt (178) und dem zweiten Abschnitt (180) des Dichtrings (174A) vorgesehen ist.

20. Eine implantierbare Schrittmacher- und/oder Defibrillationsleitung nach einem der Ansprüche 14 bis 19, wobei der erste Abschnitt (178) des Dichtrings (174A) eine abgerundete Kante entlang seines inneren Durchmessers (11) aufweist.

21. Eine implantierbare Schrittmacher- und/oder Defibrillationsleitung nach einem der Ansprüche 14 bis 20, wobei das Dichtungsmittel (174) aus einem einzigen Stück integral geformt ist.

22. Eine implantierbare Schrittmacher- und/oder Defibrillationsleitung nach einem der Ansprüche 14 bis 21, wobei das Dichtungsmittel (174) aus Gummi hergestellt ist, insbesondere aus Silikon, und eine Shore-Härte zwischen 50 und 65 Shore A aufweist.

23. Eine implantierbare Schrittmacher- und/oder Defibrillationsleitung nach Anspruch 1, umfassend:
- ein Dichtungsmittel (174) zum Abdichten des distalen Gehäuseteils (122) vom distalen Ende (138A) der Leitung (100),
wobei das Dichtungsmittel (174) einen Dichtring (174A) umfasst, der mindestens zwei Abschnitte (178, 180) in Bezug auf eine zentrale Längsachse (B) des Dichtrings (174A) aufweist,
ein erster Abschnitt (178) des Dichtrings (174A) der um die leitfähige Antriebswelle (24, 120) angeordnet ist, und ein zweiter Abschnitt (180) des Dichtrings (174A) der um den röhrenförmigen Körper (138) angeordnet ist,
wobei der erste Abschnitt (178) des Dichtrings (174A) einen äußeren Durchmesser (E1) aufweist, der strikt kleiner ist als ein innerer Durchmesser (L1) des distalen Gehäuseteils (122),
und der erste Abschnitt (178) des Dichtrings (174A) einen inneren Durchmesser (11) aufweist, der strikt kleiner ist als ein äußerer Durchmesser (E3) der leitfähigen Antriebswelle (24, 120),
und der zweite Abschnitt (180) des Dichtrings (174A) einen äußeren Durchmesser (E2) aufweist, der strikt größer ist als der innere Durchmesser (L1) des distalen Gehäuseteils (122).

## Revendications

1. Sonde de stimulation et/ou de défibrillation implantable, ladite sonde (10, 100, 200) s'étendant depuis une extrémité proximale jusqu'à une extrémité distale (114), dans laquelle l'extrémité proximale de la sonde (10, 100, 200) est configurée pour être reliée à un dispositif médical implantable, la sonde (10, 100, 200) comprenant :
- une électrode de fixation active (22, 126, 226) au niveau de l'extrémité distale (114) de la sonde (10, 100, 200), qui est couplée mécaniquement et électriquement à un arbre d'entraînement conducteur (24, 120, 220) disposé de manière mobile dans une portion distale de boîtier (122, 222) de la sonde (10, 100, 200),
dans laquelle l'électrode de fixation active (22, 126, 226) est mobile depuis une position rétractée, dans laquelle l'électrode de fixation active (22, 126, 226) est logée dans son entièreté à l'intérieur de la portion distale de boîtier (122, 222), jusqu'à une position étendue, dans laquelle l'électrode de fixation active (126, 226) s'étend dans une direction distale au moins partiellement au-delà de la portion distale de boîtier (122, 222) ; et
- un ressort de compression électriquement conducteur (176, 276),
ladite sonde (10, 100, 200) comprenant en outre :
- un élément de guidage (35, 136, 236) disposé à l'intérieur de la portion distale de boîtier (122, 222) et comprenant un corps tubulaire (138, 238) relié électriquement à la portion distale de boîtier (122, 222),
dans laquelle l'arbre d'entraînement conducteur (24, 120, 220) est disposé de manière coulissante à travers une lumière (140) qui s'étend à travers le corps tubulaire (138, 238),
dans laquelle le corps tubulaire (138, 238) comprend en outre une ouverture radiale (158, 258) en communication avec la lumière (140), et
dans laquelle le ressort de compression électriquement conducteur (176, 276) est disposé de manière partiellement radiale autour du corps tubulaire (138, 238) et de son ouverture radiale (158, 258), de telle sorte que le ressort de compression électriquement conducteur (176, 276) soit forcé par sa force de rappel contre l'arbre d'entraînement conducteur (24, 120, 220).

2. Sonde de stimulation et/ou de défibrillation implantable selon la revendication 1, dans laquelle le ressort de compression électriquement conducteur (176, 276) est un ressort hélicoïdal à bobine fermée (176, 276).

3. Sonde de stimulation et/ou de défibrillation implantable selon la revendication 1, dans laquelle le ressort de compression électriquement conducteur (176, 276) est un ressort hélicoïdal à bobine ouverte (176, 276) comprenant deux extrémités libres.

4. Sonde de stimulation et/ou de défibrillation implantable selon la revendication 1 ou 2, dans laquelle au moins une portion (162) du corps tubulaire (138) pourvue de l'ouverture radiale (158) présente un rayon extérieur (R1) qui est supérieur à un rayon intérieur (d1) du ressort de compression électriquement conducteur (716) à son état de repos.

5. Sonde de stimulation et/ou de défibrillation implantable selon l'une des revendications précédentes, dans laquelle l'ouverture radiale (158, 258) présente un angle d'ouverture (O) compris entre 100° et 250°, en particulier entre 150° et 210°, dans une section transversale du corps tubulaire (138, 238) qui est perpendiculaire à une direction d'extension (D) de la sonde (100, 200).

6. Sonde de stimulation et/ou de défibrillation implantable selon l'une des revendications précédentes, dans laquelle l'ouverture radiale (158, 258) présente une largeur (L4) le long d'une direction d'extension (D) de la sonde (100, 200) qui est supérieure à l'extension (L6) du ressort de compression électriquement conducteur (158, 258) dans cette direction (D).

7. Sonde de stimulation et/ou de défibrillation implantable selon l'une des revendications précédentes, dans laquelle le diamètre intérieur (L1) de la portion distale de boîtier (122) est supérieur au diamètre (D2) du ressort de compression électriquement conducteur (158) disposé de manière au moins partiellement radiale autour du corps tubulaire (138, 238) et de son ouverture radiale (158, 258).

8. Sonde de stimulation et/ou de défibrillation implantable selon l'une des revendications précédentes, dans laquelle le corps tubulaire (138, 238) présente au moins un bord chanfreiné (160) au niveau de l'ouverture radiale (158, 258).

9. Sonde de stimulation et/ou de défibrillation implantable selon l'une des revendications précédentes,
dans laquelle le corps tubulaire (138) comprend au moins :
- une première portion (162) pourvue de l'ouverture radiale (158) et d'un élément (37, 142, 144) du corps tubulaire (138) en contact avec la portion distale de boîtier (122) ; et
- une deuxième portion (164) qui s'étend depuis une extrémité distale (138A) du corps tubulaire (138) jusqu'à la première portion (162),
et dans laquelle un moyen de scellement (174) est disposé au niveau de la deuxième portion (164) afin de sceller la première portion (162) du corps tubulaire (138) à partir de l'extrémité distale (138A) de la sonde (100).

10. Sonde de stimulation et/ou de défibrillation implantable selon l'une des revendications précédentes, dans laquelle le corps tubulaire (138) de l'élément de guidage (136) est relié mécaniquement et électriquement à la portion distale de boîtier (22) par le biais d'une soudure.

11. Procédé d'assemblage d'une sonde de stimulation et/ou de défibrillation implantable (10, 100, 200) selon l'une des revendications 1 à 10, comprenant les étapes consistant à :
a) disposer le ressort de compression électriquement conducteur (176, 276) autour du corps tubulaire (138, 238) de l'élément de guidage (136, 236) au niveau de l'ouverture radiale (158, 258), de telle sorte qu'une portion (176B) du ressort de compression électriquement conducteur (176, 276) soit partiellement déviée à l'intérieur de la lumière (140) en raison de sa force de rappel (F) ; puis
b) insérer l'arbre d'entraînement conducteur (120, 220) à travers la lumière (140) de l'élément de guidage conducteur (136, 236), poussant ainsi partiellement le ressort de compression (176, 276) à l'extérieur de la lumière (140) de manière à établir une connexion électrique entre le ressort de compression électriquement conducteur (176, 276) et l'arbre d'entraînement conducteur (120, 220) ; et puis
c) relier mécaniquement et électriquement le corps tubulaire (138, 238) de l'élément de guidage (136, 236) à la portion distale de boîtier (122, 222) de la sonde (100, 200).

12. Procédé selon la revendication 11, dans lequel l'étape b) consiste en outre à disposer d'un moyen de scellement (174) au niveau de l'extrémité distale (138A, 162) du corps tubulaire (138) afin de le sceller à partir de l'extrémité distale (114) de la sonde (100).

13. Procédé selon la revendication 12, dans lequel l'étape c) consiste à souder le corps tubulaire (138, 238) à la portion distale de boîtier (122, 222) de la sonde (100, 200), en particulier par soudage laser.

14. Sonde de stimulation et/ou de défibrillation implantable, ladite sonde (10, 100) s'étendant depuis une extrémité proximale jusqu'à une extrémité distale (114), dans laquelle l'extrémité proximale de la sonde (10, 100) est configurée pour être reliée à un dispositif médical implantable, la sonde (10, 100) comprenant :
- une électrode de fixation active (22, 126) au niveau de l'extrémité distale (114) de la sonde (10, 100), qui est couplée mécaniquement et électriquement à un arbre d'entraînement conducteur (24, 120) disposé de manière mobile dans une portion distale de boîtier (122) de la sonde,
dans laquelle l'électrode de fixation active (126) est mobile depuis une position rétractée, dans laquelle l'électrode de fixation active (22, 126) est logée dans son entièreté à l'intérieur de la portion distale de boîtier (122), jusqu'à une position étendue, dans laquelle l'électrode de fixation active (126) s'étend dans une direction distale au moins partiellement au-delà de la portion distale de boîtier (122),
ladite sonde (10, 100) comprenant en outre :
- un élément de guidage (35, 136) disposé à l'intérieur de la portion distale de boîtier (122) et comprenant un corps tubulaire (138) relié électriquement à la portion distale de boîtier (122), dans laquelle l'arbre d'entraînement conducteur (24, 120) est disposé de manière coulissante et rotative à travers une lumière (140) qui s'étend à travers le corps tubulaire (138) ; et
- un moyen de scellement (174) servant à sceller la portion distale de boîtier (122) à partir de l'extrémité distale (138A) de la sonde (100),
le moyen de scellement (174) comprenant une bague d'étanchéité (174A) ayant au moins deux portions (178, 180) par rapport à un axe longitudinal central (B) de ladite bague d'étanchéité (174A), une première portion (178) de la bague d'étanchéité (174A) étant disposée autour de l'arbre d'entraînement conducteur (24, 120), et une deuxième portion (180) de la bague d'étanchéité (174A) étant disposée autour du corps tubulaire (138),
la première portion (178) de la bague d'étanchéité (174A) ayant un diamètre externe (E1) qui est strictement inférieur à un diamètre interne (L1) de la portion distale de boîtier (122), et
la première portion (178) de la bague d'étanchéité (174A) ayant un diamètre interne (11) qui est strictement inférieur à un diamètre externe (E3) de l'arbre d'entraînement conducteur (24, 120), et
la deuxième portion (180) de la bague d'étanchéité (174A) ayant un diamètre externe (E2) qui est strictement supérieur au diamètre interne (L1) de la portion distale de boîtier (122),
dans laquelle une extrémité distale (138A) du corps tubulaire (138) est pourvue d'un épaulement externe faisant saillie radialement (168), ledit épaulement externe faisant saillie radialement (168) étant ajusté par forme dans un renfoncement radial et interne correspondant (184) de la bague d'étanchéité (174A) du moyen de scellement (174).

15. Sonde de stimulation et/ou de défibrillation implantable selon la revendication 14, dans laquelle la différence de diamètre en pourcentage entre le diamètre externe (E1) de la première portion (178) de la bague d'étanchéité (174A) et le diamètre interne (L1) de la portion distale de boîtier (122) est comprise entre 3 % et 30 %, en particulier entre 5 % et 20 %.

16. Sonde de stimulation et/ou de défibrillation implantable selon la revendication 14 ou 15, dans laquelle la différence en pourcentage entre le diamètre interne (11) de la première portion (178) de la bague d'étanchéité (174A) et un diamètre externe (E3) de l'arbre d'entraînement conducteur (120) est comprise entre 0,05 % et 20 %, en particulier entre 1 % et 10 %.

17. Sonde de stimulation et/ou de défibrillation implantable selon la revendication 14, 15 ou 16, dans laquelle la différence en pourcentage entre le diamètre externe (E2) de la deuxième portion (180) de la bague d'étanchéité (174A) et le diamètre interne (L1) de la portion distale de boîtier (122) est comprise entre 0,5 % et 20 %, en particulier entre 1 % et 10 %.

18. Sonde de stimulation et/ou de défibrillation implantable selon l'une des revendications 14 à 16, dans laquelle le diamètre interne (11) de la première portion (178) de la bague d'étanchéité (174A) est inférieur au diamètre interne (12) de la deuxième portion (180) de la bague d'étanchéité (174A).

19. Sonde de stimulation et/ou de défibrillation implantable selon la revendication 14, dans laquelle le renfoncement radial et interne (184) de la bague d'étanchéité (174A) est disposée au niveau d'une jonction (179) entre la première portion (178) et la deuxième portion (180) de la bague d'étanchéité (174A).

20. Sonde de stimulation et/ou de défibrillation implantable selon l'une des revendications 14 à 19, dans laquelle la première portion (178) de la bague d'étanchéité (174A) présente un bord arrondi le long de son diamètre interne (11).

21. Sonde de stimulation et/ou de défibrillation implantable selon l'une des revendications 14 à 20, dans laquelle le moyen de scellement (174) est formé d'un seul tenant en une seule pièce.

22. Sonde de stimulation et/ou de défibrillation implantable selon l'une des revendications 14 à 21, dans laquelle le moyen de scellement (174) est réalisé en caoutchouc, en particulier en silicone, et présente une dureté Shore comprise entre 50 et 65 Shore A.

23. Sonde de stimulation et/ou de défibrillation implantable selon la revendication 1, comprenant en outre :
- un moyen de scellement (174) servant à sceller la portion distale de boîtier (122) de l'extrémité distale (138A) de la sonde (100),
le moyen de scellement (174) comprenant une bague d'étanchéité (174A) ayant au moins deux portions (178, 180) par rapport à un axe longitudinal central (B) de ladite bague d'étanchéité (174A), une première portion (178) de la bague d'étanchéité (174A) étant disposée autour de l'arbre d'entraînement conducteur (24, 120), et une deuxième portion (180) de la bague d'étanchéité (174A) étant disposée autour du corps tubulaire (138),
la première portion (178) de la bague d'étanchéité (174A) ayant un diamètre externe (E1) qui est strictement inférieur à un diamètre interne (L1) de la portion distale de boîtier (122), et
la première portion (178) de la bague d'étanchéité (174A) ayant un diamètre interne (11) qui est strictement inférieur à un diamètre externe (E3) de l'arbre d'entraînement conducteur (24, 120), et
la deuxième portion (180) de la bague d'étanchéité (174A) ayant un diamètre externe (E2) qui est strictement supérieur au diamètre interne (L1) de la portion distale de boîtier (122).
